# EUROPEAN PATENT SPECIFICATION

(11) **EP 4 165 406 B1**
(45) Date of publication and mention of the grant of the patent: **02.12.2026**
(21) Application number: 21734528.9
(22) Date of filing: 10.06.2021
(51) Int. Cl.: G01N 33/53, G01N 33/543, B01L 3/00, C12M 1/26, C12M 1/28

(54) **SALIVA TREATMENT DEVICES**
SPEICHELBEHANDLUNGSGERÄTE
DISPOSITIFS DE TRAITEMENT DE LA SALIVE

(30) Priority: 10.06.2020 US 202063037096 P; 30.11.2020 US 202063119018 P
(43) Date of publication of application: 19.04.2023
(73) Proprietor: Salignostics Ltd., Jerusalem (IL)
(72) Inventor: DEUTSCH, Omer, 9062700 Ofra (IL); COHEN, Raluca, 9262227 Jerusalem (IL); NEUMANN, Yoav, 9987500 Tzur Hadassah (IL); KRIEF, Guy, 9654415 Jerusalem (IL)
(74) Representative: Evens, Paul Jonathan
(86) International application number: PCT/IL2021/050697
(87) International publication number: WO 2021/250672

(56) References cited:
- WO-A1-2007/062575
- WO-A1-2008/131033
- WO-A1-2016/029020
- WO-A1-2019/159167
- CN-A- 105 985 950
- US-A1- 2003 064 526
- US-A1- 2008 076 169
- US-A1- 2008 118 397
- US-A1- 2009 143 699
- ZHANG YI ET AL: "A stacking flow immunoassay for the detection of dengue-specific immunoglobulins in salivary fluid", LAB ON A CHIP, vol. 15, no. 6, 1 January 2015 (2015-01-01), UK, pages 1465 - 1471, XP055873249, ISSN: 1473-0197, Retrieved from the Internet <URL:https://pubs.rsc.org/en/content/articlepdf/2015/lc/c4lc01127a> DOI: 10.1039/C4LC01127A

## Description

### CROSS-REFERENCE TO RELATED APPLICATIONS

The present application claims priority from US Provisional application 63/037,096 to Cohen et al., entitled "LATERAL FLOW SYSTEM," which was filed on June 10, 2020, and US Provisional application 63/119,018 to Deutsch et al., entitled "BODY FLUID TREATMENT DEVICE," which was filed on November 30, 2020.

### FIELD OF THE INVENTION

Some applications of the present invention generally relate to medical apparatus. Specifically, the present invention relates to apparatus for use by a subject, e.g. for detecting analytes in biological samples such as saliva.

### BACKGROUND

Diagnostic assays are performed on samples of body fluids taken from a subject, in order to detect the presence and/or concentration of various analytes in the sample, and to thereby determine a condition of the subject. It is important that such assays be sufficiently sensitive and reliable. For some conditions, such assays are performed frequently, such that accessibility and convenience are important.

For some diagnostic assays, there may be a trade-off between accessibility and convenience on one hand, and sensitivity and reliability on the other hand. For example, it may be convenient for the untrained user to collect the sample (e.g., for the subject to collect the sample him/herself), independently of laboratory-based methods. One impediment to the use of such samples is the need to preserve the integrity (e.g., the molecular integrity, such as antigenicity) of the collected sample while it is stored and/or transported to a diagnostic laboratory. Specifically, diagnostic assays frequently require that samples be kept in temperature-controlled conditions until the samples are used in a diagnostic assay.

Diagnostic assays designed for home or point-of-care testing (e.g., self-testing) are often more convenient and accessible to the subject being tested. However, laboratory-based methods for detecting analytes in body fluid samples are frequently more reliable than self-testing methods. This may be due in part to samples requiring processing that is typically performed by skilled technicians in a laboratory setting.

Factors affecting the sensitivity and reliability, as well as the accessibility and convenience of self-testing diagnostic assays include the means by which: (i) the body fluid sample is collected, (ii) the fluid sample is processed, and (iii) the analyte is detected.

WO 2019/159167 discloses apparatus according to the pre-amble of appended independent claim 1. WO 2019/159167 and family member US Patent Application Publication 2020-0386752 to Deutsch et al., describes a barrel which defines a channel, and has an opening into the channel, and an outlet from the channel. A porous carrier disposed within the channel carries an albumin. A cellulosic stationary phase is disposed within the channel between the carrier and the distal region of the barrel. A lateral flow platform is coupled to the barrel such that a sample pad of the lateral flow platform is in fluid communication with the outlet. A sponge, coupled to a plunger, is configured to hold saliva. The plunger is dimensioned to compress the sponge within the channel such that the saliva is driven (i) out of the sponge and through the carrier, dissolving at least some of the albumin, (ii) with the dissolved albumin, into the stationary phase, and (iii) as an eluate, out of the stationary phase, through the outlet, and onto the sample pad. Other embodiments are also described.

WO 2008/131033 relates to a sample collector.

US 2008/181397 relates to a specimen sample collection device and test system.

WO 2016/029020 relates to devices, solutions and methods for sample collection related applications.

US 2003/064526 relates to a sample collector and test device, and discloses a device for detecting analytes in oral fluid which comprises a sample collector module reversibly attached to a test module housing a lateral flow test strip.

### SUMMARY OF THE INVENTION

In accordance with the present invention, there is provided apparatus as defined in appended independent claim 1. Embodiments of the present invention are defined in appended claims dependent on independent claim 1.

Applications of the present disclosure are directed to collection of a sample of saliva from a subject by an untrained user. Typically, an absorbent sponge is placed in the mouth of the subject, where the sponge absorbs the saliva. Some applications of the present invention are directed to treatment of saliva by the untrained user in a manner that facilitates preservation of the saliva until it is used in a diagnostic assay. Typically, the unskilled user dissolves a surfactant into the sample, in such a way that preserves the saliva while it is transported, at room temperature, to a diagnostic laboratory.

For some such applications, a system includes a sponge for collecting the saliva, and a receptacle in which the sample is processed. The receptacle defines a channel within which a porous carrier that holds a surfactant is disposed. The sponge and the channel are configured such that the unskilled user may easily compress the sponge within the channel, such that the saliva is driven: (i) out of the sponge and into the carrier, (ii) through the carrier, such that the surfactant is dissolved into the saliva, and (iii) with the dissolved surfactant, into a collection tube. The saliva with the dissolved surfactant may then be stored at room temperature (e.g., for up to 72 hours) until the saliva is used in a diagnostic assay.

Some applications of the present disclosure are directed to determining a condition of the subject by performing (e.g., by the untrained user, outside of a laboratory setting) a diagnostic assay using the saliva.

For some applications, a system includes a sponge for collecting the saliva, a receptacle in which the saliva is processed, and a downstream diagnostic assay. The receptacle defines a channel within which a porous carrier that holds dry buffer components is disposed. Typically for such applications, the diagnostic assay is in fluid communication with the channel. Further typically, the system is configured such that the unskilled user may easily compress the sponge within the channel, such that the saliva is driven: (i) out of the sponge and into the carrier, (ii) through the carrier, such that the dry buffer components are dissolved into the saliva, and (iii) with the dissolved buffer components, into the diagnostic assay.

For some applications, the sponge is coupled to a plunger that is shaped to define a plunger screw-thread, and the receptacle is shaped to define a receptacle screw-thread. Typically for such applications, the unskilled user compresses the sponge within the channel by screwing the plunger screw-thread along the receptacle screw-thread. For some such applications, one of the screw-threads defines a protrusion that generates greater resistance during screwing of the plunger, than do other portions of the screw-thread. This greater resistance may be felt by the user when the protrusion engages the other screw-thread, thereby aiding the user in regulating the distance to which the sponge is advanced through the channel.

For some such applications, the system further comprises a stationary phase (e.g., a cellulosic stationary phase) that is disposed within the channel, between the carrier and the diagnostic assay. Typically for such applications, the system is configured such that the unskilled user may easily compress the sponge within the channel, such that the saliva is driven: (i) out of the sponge and into the carrier, (ii) through the carrier, such that the dry buffer components are dissolved into the saliva, (iii) with the dissolved buffer components, into the stationary phase, and (iv) as an eluate that is eluted from the saliva with the dissolved buffer components, out of the stationary phase and into the diagnostic assay.

For some applications, the diagnostic assay is a lateral flow assay. For some such applications, and in accordance with embodiments of the present invention, the receptacle defines a flow-regulation component configured to optimize flow of the saliva from the channel into the lateral flow assay, e.g., into a sample pad thereof. For such applications, the saliva flows out from the channel and into a chamber defining a pool. Once the saliva reaches the chamber, the saliva flows into the pool and soaks into the sample pad, from where the saliva passes through the lateral flow assay via capillary action. In this way, the flow-regulation component ensures that a desired volume of the saliva is introduced to the assay, thereby facilitating self-testing that is sensitive and reliable.

For some such applications, the flow-regulation component comprises a drainage hole that reduces a risk of too large a volume of saliva entering the lateral flow assay. For some applications, the system further comprises a catchment tray that is in fluid communication with the drainage hole. In this way, the user is less likely to come into contact with excess saliva that is drained from the lateral flow platform.

For some applications, the absorbent sponge has a capacity for a desired volume of saliva, and comprises an indicator that indicates (e.g., visually) that the sponge holds the desired volume of saliva. Alternatively or in addition, the system may comprise a scale for determining whether a predetermined amount of saliva has been collected into the sponge. In this way, the untrained user can reliably and conveniently collect the saliva.

There is therefore provided, in accordance with the present invention, an apparatus, for use by a subject, the apparatus including:
a sampler, including:
   a sponge, configured to be placed in a mouth of the subject, and to absorb a sample of saliva from the mouth;
   a plunger, having a distal portion that is coupled to the sponge;
a lateral flow platform, including a sample pad; and
a casing, housing the lateral flow platform, and including:
   a channel, having:
      an opening into the channel at a proximal region of the channel, and
      an outlet from the channel at a distal region of the channel, the sampler being configured to, while the sponge holds the sample, introduce the sponge into the channel via the opening, the plunger being dimensioned to slide snugly distally within the channel in a manner that compresses the sponge and pushes
   the sample out of the sponge and through the outlet;
characterized in that the casing further comprises:
   a chamber having a floor, the floor defining an aperture therethrough, at least a portion of the sample pad disposed below the aperture such that the aperture and the portion of the sample pad collectively define a pool, the aperture defining a rim of the pool, and the portion of the sample pad defining a bottom of the pool; and
   a nozzle, extending from the outlet, and opening into the chamber in an orientation that directs the sample from the outlet toward the rim of the pool.

In an application, the lateral flow platform includes a human chorionic gonadotropin (HCG) lateral flow test including antibodies specific to HCG.

In an application, the casing is configured to allow elevated pressure in the chamber, resulting from the plunger sliding snugly distally within the channel, to be released to outside of the casing.

In an application, the casing defines a barrel that is shaped to define the channel.

In an application, the apparatus includes (i) a porous carrier, disposed within the channel proximally from the outlet, and (ii) one or more dry buffer components, held in the carrier, at least one of the buffer components being a surfactant held in the carrier, and:
the plunger is dimensioned to slide snugly distally within the channel in a manner that compresses the sponge and pushes the sample:
out of the sponge and into the carrier, dissolving at least some of the buffer components, and
out of the carrier with the dissolved buffer components, and through the outlet.

In an application, the carrier includes a plurality of fibers arranged such that:
spaces between the fibers define pores of the carrier, and
the fibers are generally not arranged along a longitudinal axis of the carrier.

In an application, the apparatus does not include a cellulosic stationary phase.

In an application, the apparatus does not include a stationary phase.

In an application, the apparatus does not include an albumin.

In an application, the apparatus does not include a protein.

In an application, the apparatus includes a stationary phase disposed within the channel proximally from the outlet, and the plunger is dimensioned to slide snugly distally within the channel in a manner that compresses the sponge and pushes the sample out of the sponge, through the stationary phase, and as an eluate that is eluted from the saliva, out of the stationary phase and through the outlet.

In an application, the apparatus includes (i) a porous carrier, disposed within the channel proximally from the stationary phase, and (ii) one or more dry buffer components, held in the carrier, at least one of the buffer components being a surfactant, and:
the plunger is dimensioned to slide snugly distally within the channel in a manner that compresses the sponge and pushes the sample:
out of the sponge and into the carrier, dissolving at least some of the buffer components,
out of the carrier with the dissolved buffer components, through the stationary phase, and
as an eluate that is eluted from the saliva with the dissolved buffer components, through the outlet.

In an application, the carrier includes a plurality of fibers arranged such that:
spaces between the fibers define pores of the carrier, and
the fibers are generally not arranged along a longitudinal axis of the carrier.

In an application, the apparatus does not include an albumin.

In an application, the apparatus does not include a protein.

In an application, the apparatus includes a protein, held in the carrier, the apparatus configured such that sliding the plunger distally within the channel compresses the sponge and pushes the sample:
out of the sponge and into the carrier, dissolving at least some of the protein and some of the buffer components,
out of the carrier with the dissolved protein and the dissolved buffer components, through the stationary phase, and
as an eluate that is eluted from the saliva with the dissolved buffer components and the dissolved protein, through the outlet.

In an application:
the nozzle defines a nozzle axis,
the channel is disposed at a proximal portion of the casing that is proximal to the nozzle,
the nozzle is oriented to direct the sample from the outlet distally toward the rim of the pool, and
the sample pad is disposed below the nozzle axis.

In an application, the chamber has a hollow volume, and most of the hollow volume is disposed above the nozzle axis.

In an application, the floor slopes distally upward.

In an application, a proximal part of the floor is disposed below the nozzle axis, and the floor slopes distally upward to intersect the nozzle axis.

In an application, the floor slopes distally upward at 1-10 degrees with respect to the nozzle axis.

In an application, the floor slopes distally upward at 1-5 degrees with respect to the nozzle axis.

In an application, the floor slopes distally upward at 2-4 degrees with respect to the nozzle axis.

In an application, the floor slopes distally upward at 3 degrees with respect to the nozzle axis.

In an application, the channel is colinear with the nozzle.

In an application, the sample pad is parallel with the nozzle axis.

In an application, the casing defines a spillway, and the floor defines one or more overflow holes therethrough, the one or more overflow holes and the spillway configured to direct excess sample out of the chamber via the one or more overflow holes to the spillway.

In an application, the casing is shaped to define a plurality of cavities, the cavities being disposed in series proximally of the spillway, and
each pair of consecutive cavities are separated from each other by a baffle that is configured to allow limited fluid communication between the pair of consecutive cavities.

In an application, the spillway defines a drainage hole.

In an application, the drainage hole is disposed proximally of the sample pad.

In an application, the apparatus includes a catchment tray, the catchment tray being in fluid communication with the drainage hole.

In an application, at least part of the spillway is disposed below at least part of the lateral flow platform.

In an application, at least the part of the spillway is disposed below the sample pad.

In an application, the one or more overflow holes are disposed proximally from the aperture.

In an application, the one or more overflow holes include a first overflow hole and a second overflow hole, the first and second overflow holes disposed on either side of a direct path between the nozzle and the aperture.

In an application, the first and second overflow holes are configured to facilitate directing of the sample distally from the nozzle toward the rim of the pool by inhibiting the sample from dispersing laterally.

The present invention will be more fully understood from the following detailed description of applications thereof, taken together with the drawings, in which:

### BRIEF DESCRIPTION OF THE DRAWINGS

Figs. 1 and 2A-E are schematic illustrations showing a system for use with a body fluid (e.g., saliva) of a subject;
Fig. 3 is a flowchart that schematically illustrates at least some steps of a method that is performed in accordance with some applications of the present disclosure;
Figs. 4A-B show results of experiments performed on untreated saliva, or on saliva treated according to an application of the disclosure;
Figs. 5A-B, 6, 7 and 8 are schematic illustrations of a system for use with saliva of a subject, in accordance with some applications of the invention;
Figs. 9-10 are schematic illustrations of a system for use with saliva of a subject;
Figs. 11A-C, 12 and 13A-C are schematic illustrations of a system for use with saliva of a subject; and
Figs. 14-15 show results of experiments conducted using a system for use with saliva of a subject.

### DETAILED DESCRIPTION OF EMBODIMENTS

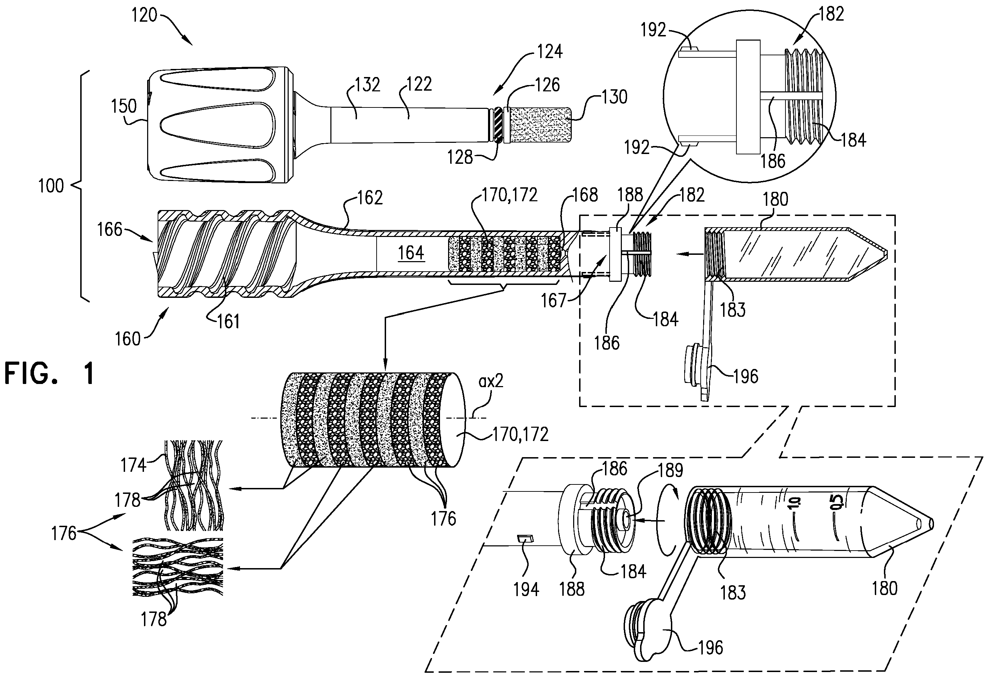

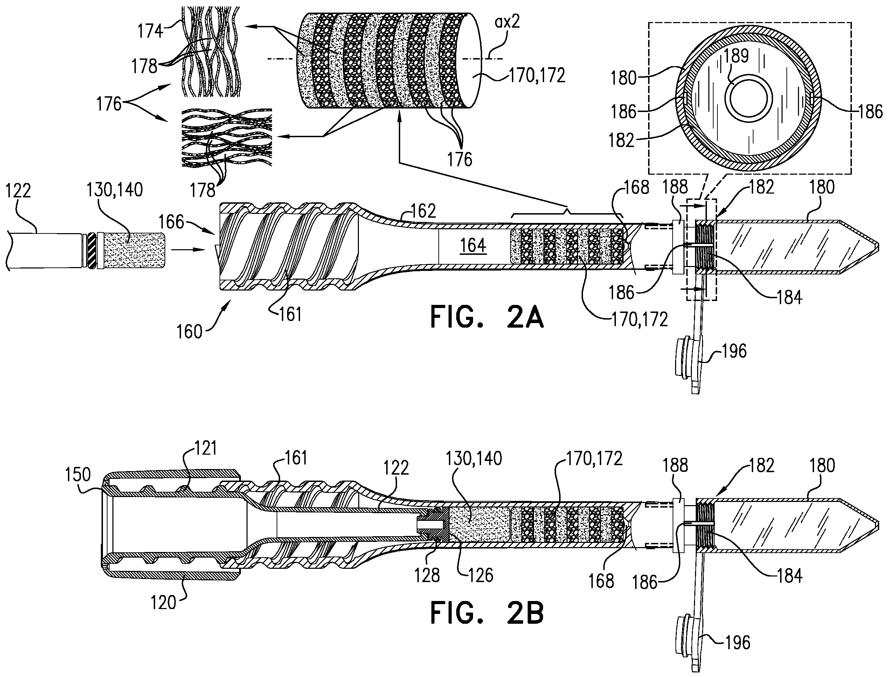

Reference is made to Figs. 1 and 2A-E, which are schematic illustrations of a system 100 for use with a body fluid (e.g., saliva 140) of a subjectn.

Although applications of the invention are described hereinbelow as being used with saliva 140, some applications may be suitable for use with other body fluids (e.g., urine or blood), *mutatis mutandis.*

System 100 comprises an elongate barrel 162 and a plunger 122. Typically, plunger 122 is defined by, or is a component of, a first structure 120 of system 100. Typically, barrel 162 is defined by, or is a component of, a second structure 160 of system 100. Each of structures 120 and 160 typically comprises several integrated components, e.g., that are fixed with respect to each other. Typically, structures 120 and 160 are separate from each other (e.g., are provided uncoupled to each other), and are coupled to each other by the user during normal use.

As shown in Fig. 1, barrel 162 is shaped to define a channel 164 therethrough. Alternatively, some applications of the invention may be performed using alternate receptacles that define a channel. Such channels typically have an upstream opening and a downstream outlet ("upstream" and "downstream" relating to the direction of flow of saliva 140 through the channel, as described hereinbelow). The description hereinbelow of structure 160 is not meant to exclude such alternate receptacles that define a channel, *mutatis mutandis.*

As shown, barrel 162 defines an opening 166 into channel 164 at a proximal (i.e., upstream) region of the barrel, and an outlet 168 from the channel at a distal (i.e., downstream) region of the barrel. For some applications, and as shown, barrel 162 is shaped to define a distal opening 167 (e.g., further distally of outlet 168).

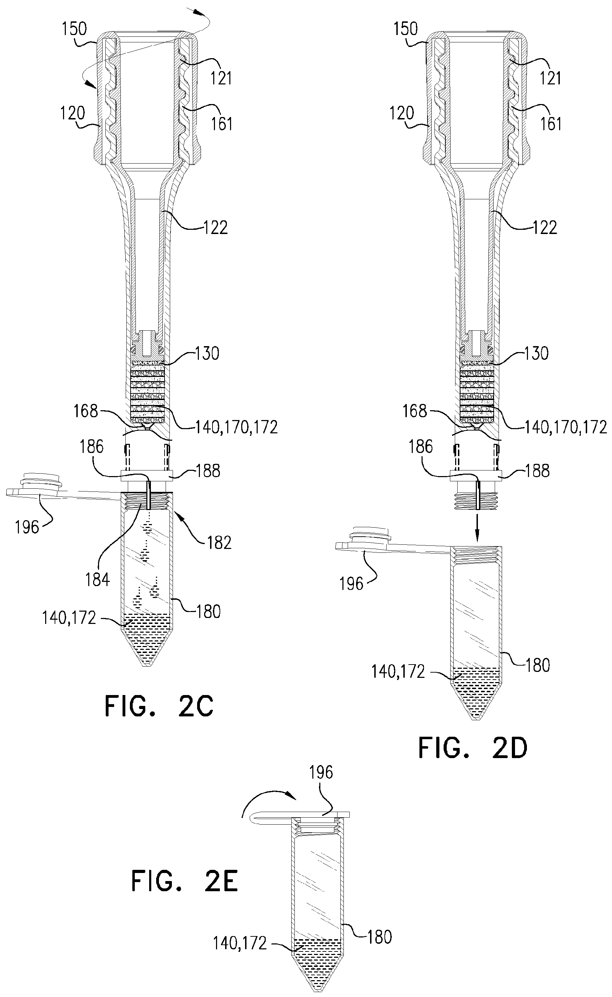

For some applications and as shown, structure 120 (e.g., plunger 122 thereof) (i) comprises a screw thread 121 at a proximal portion, configured to couple the plunger to structure 160 (e.g., barrel 162 thereof), and (ii) is dimensioned such that coupling (e.g., screwing) the plunger to the barrel using the screw thread compresses a sponge 130 within channel 164 by sliding distal portion 124 of the plunger through the channel. This is illustrated by the helical arrow in Fig. 2C. For such applications, structure 160 (e.g., barrel 162 thereof) typically comprises a corresponding screw thread 161. Structure 120 (e.g., plunger 122 thereof) typically comprises a stem 132 that extends between screw thread 121 and distal portion 124.

Typically, a porous carrier 170 is disposed within channel 164 (e.g., between opening 166 and outlet 168, as shown). Further typically, a width of carrier 170 is such that the carrier fits snugly within channel 164. For some applications, the width of carrier 170 is between 7 and 12 mm.

Further typically, a length of carrier 170 is such that the carrier fits within channel 164 of barrel 162. For some applications, the length of carrier 170 is sized in order to allow for at least part of sponge 130 to enter channel 164 without requiring compression of sponge 130 and/or carrier 170. For some such applications, the length of carrier 170 is between 5 and 15 mm.

For some applications, carrier 170 comprises a plurality of fibers 174 (e.g., synthetic fibers, such as polyolefin fibers), and spaces between the fibers define pores 178 within the carrier.

Typically for such applications, fibers 174 are not arranged along a carrier axis ax2 of carrier 170. Instead, fibers 174 may be arranged orthogonally to carrier axis ax2 of carrier 170. For example, and as shown in the left inset of Figs. 1 and 2A, carrier 170 may comprise adjacent layers of fibers 174. As shown, fibers 174 of each layer are rotationally offset to the fibers of the adjacent layers (e.g., are perpendicular to the fibers of the adjacent layers). Typically, carrier 170 is disposed snugly fitted within channel 164.

Typically, a surfactant 172 is held in carrier 170 (e.g., the carrier is impregnated with the surfactant). For example, surfactant 172 may comprise polysorbate 20, polyethylene glycol nonylphenyl ether, 3-[(3-cholamidopropyl)dimethylammonio]-1-propanesulfonate, sodium dodecyl sulfate, ethyl trimethyl ammonium bromide and/or an octylphenol ethoxylate.

For some applications, surfactant 172 is a component of a buffer, such as a lysis buffer (e.g., a commercially available lysis buffer such as DNA/RNA Shield (TM) manufactured by Zymo Research (Irvine, CA, USA)). For some such applications, the protease inhibitor is also a component of the buffer. Typically for such applications, between 50 and 200 mg of dry buffer components are held in carrier 170. That is, the buffer components are held in carrier 170 in such a quantity that, when between 500-750 microliters of saliva dissolve the lysis buffer components, the resulting solution is an active lysis buffer solution (i.e., a lysis buffer solution that ruptures cellular membranes).

Typically, and as shown, system 100 (e.g., structure 120 thereof) comprises sponge 130 that is coupled to a distal portion 124 of plunger 122. For some applications, and as shown, distal portion 124 of plunger 122 defines a crown 126 at a distal end of the plunger, and a piston ring 128, e.g., proximal from the crown. For such applications, sponge 130 is disposed distally from crown 126, e.g., attached to a distal face of the crown.

Sponge 130 is configured to hold a fluid, such as saliva 140, and structure 120 (e.g., plunger 122 thereof) is configured to introduce the sponge holding the saliva into channel 164 via opening 166 (Fig. 2B). Typically, saliva 140 is introduced into sponge 130 by bathing the sponge in the saliva, such that the sponge absorbs the saliva. For some applications, sponge 130 is configured to hold between 200 and 800 microliters of saliva.

Typically, for applications in which the body fluid is saliva 140, distal portion 124 of plunger 122 is placed in the mouth of the subject, where sponge 130 absorbs the saliva. For such applications, sponge 130 is therefore configured to be safe for placement in the mouth of the subject. For example, sponge 130 is securely attached to plunger 122, is non-toxic, and/or is sterile. Typically, sponge 130 contains no additional substances therein that may release and/or dissolve in the mouth of the subject. For some applications, sponge 130 comprises polymeric fibers.

For some applications, sponge 130 comprises an indicator that indicates (e.g., visually) that the sponge holds a desired volume of saliva 140. For some such applications, the indicator may correspond to the indicator described in US 9,330,580 to Ward et al., which is incorporated herein by reference. For some applications, the desired volume of saliva is between 200 and 800 microliters. It is hypothesized by the inventors that the indicator may facilitate use of system 100 by an untrained user (e.g., by a patient).

Further typically, a collection tube 180 is reversibly couplable to outlet 168 of barrel 162. For some applications, collection tube 180 has a capacity that is between 100 and 2000 microliters, and, for example, may be a commercially-available PCR tube.

For some applications, collection tube 180 is integral to system 100 (i.e., is supplied together with first structure 120 and second structure 160). For some such applications, collection tube 180 is provided while the tube is coupled to outlet 168. Alternatively, collection tube 180 may be coupled to outlet 168 by a user of system 100.

For some applications, collection tube 180 may be provided separately from structures 120, 160.

For some applications, and as shown, an adapter 182 is used to couple collection tube 180 to outlet 168 (thereby facilitating transfer of saliva from channel 164, through the outlet and into collection tube 180). For some applications, adapter 182 defines a nozzle 189 to direct saliva into collection tube 180. For some applications, and as shown, adapter 182 defines an adapter-threading 184 that is complementary to a tube-threading 183, such that collection tube 180 may be screwed onto adapter 182 (lower inset of Fig. 1).

For some applications, adapter 182 is reversibly couplable to barrel 162. For example, system 100 may comprise adapters 182 of different dimensions and/or having different features (e.g., threading) to facilitate coupling of outlet 168 to collection tubes 180 of different types. For example, different adapters 182 may be used to couple outlet 168 to collection tubes 180 configured for specific diagnostic assays (e.g., Polymerase chain reaction (PCR) assay, Fluorescence-activated cell sorting (FACS), Enzyme-linked immunosorbent assay (ELISA), Immunochromatographic assays), or to collection tubes configured for cryopreservation.

For some applications, and as shown in Fig. 1, a proximal region of adapter 182 is shaped to define one or more detents 192 which are located complementarily to slots 194 in the distal region of barrel 162. Typically for such applications, when the proximal region of adapter 182 is inserted within the distal region of barrel 162, detents 192 snap into place upon entering slots 194. Alternatively or in addition, adapter 182 may be shaped to define a cuff 188 to facilitate coupling the adapter to barrel 162. In this way, users may readily manually couple an adapter 182 of their choice to barrel 162, according to the desired downstream application, as described hereinabove. For some such applications, adapter 182 may also be removed by the user (e.g., by pressing upon detents 192 from outside of slots 194, and pulling adapter 182 distally), and replaced with a different adapter of choice.

For some applications, and as shown in the upper inset of Fig. 1, adapter 182 defines a vent 186 (e.g., through adapter-threading 184) through which air may be released from the interior of collection tube 180, while the adapter couples the collection tube to outlet 168. For example, and as shown, vent 186 may be a longitudinal groove that transverses adapter-threading 184. As shown in Fig. 2A, since vents 186 extend from within collection tube 180 to outside the collection tube, e.g., to the atmosphere, the vents facilitate air flow from within the collection tube to outside of the collection tube. Release of air from within the collection tube to outside of the collection tube via vent 186 facilitates advancing plunger 122 through channel 164, by avoiding an undesirable resistance due to increased air pressure within collection tube 180.

For some applications in which system 100 does not comprise an adapter as a distinct element, the distal region of barrel 162 may be shaped similarly to the adapter, in order to facilitate direct coupling of the distal region of the barrel to collection tube 180, *mutatis mutandis.* For example, the distal region of barrel 162 may be shaped to define barrel-threading that is complementary to tube-threading 183.

Fig. 2B shows structures 120 and 160 positioned with the distal portion of plunger 122 disposed within channel 164 but prior to engagement of screw threads 121 and 161. As shown, in this state, sponge 130 typically remains largely (e.g., completely) uncompressed, e.g., such that compression of the sponge occurs primarily (e.g., only) via screwing of screw threads 121 and 161. Also as shown, in this state, the distal portion of barrel 162 is disposed sufficiently distally (i.e., deeply) within channel 164 to have sealed the channel (e.g., by piston ring 128 thereof), despite more proximal portions of the channel, closer to opening 166, being typically wider. This therefore prevents inadvertent leakage of the saliva during subsequent distal advancement of plunger 122.

Fig. 2C shows structures 120 and 160 following complete distal advancement of plunger 122, e.g., by screwing screw thread 121 as far as possible - e.g., until it reaches the end of screw thread 161, and/or until distal portion 124 of the plunger cannot be advanced further distally. As shown, once system 100 is in this state, sponge 130 has become compressed, such that saliva 140 has been squeezed out of the sponge, and through carrier 170.

Structure 120 (e.g., plunger 122 thereof) is dimensioned such that sliding of distal portion 124 through channel 164 compresses sponge 130 within the channel (Fig. 2C). Typically, sliding of distal portion 124 of plunger 122 through channel 164 compresses sponge 130 between plunger 122 and carrier 170 (e.g., pressing the sponge against the carrier - i.e., with the sponge in contact with the carrier). To facilitate this, sponge 130 is sufficiently compressible to be compressed by force applied via plunger 122. Compression of sponge 130 holding saliva 140 drives the saliva (i) out of the sponge, (ii) through carrier 170, dissolving at least some of surfactant 172 disposed therein, and (iii) through outlet 168, typically to collection tube 180. In this way, advancement of plunger 122 serves as a pressure-applying mechanism for dissolving surfactant 172 into saliva 140.

It is hypothesized by the inventors that the dimensions of carrier 170, as well as the arrangement of fibers 174 of the carrier orthogonally to carrier axis ax2 (e.g., in the matrix described hereinabove in reference to Fig. 2A), facilitates dissolving surfactant 172 into saliva 140 as the saliva is driven through carrier 170. That is, since pores 178 are to some extent randomly distributed based on the arrangement of fibers 174, saliva 140 will likely not pass through the carrier in a smooth, laminar flow. Instead, saliva 140 may pass through a pore 178 defined by one layer 176 of fibers 174, and upon reaching the next layer of fibers, the saliva will move at least partially laterally, until reaching a pore defined by fibers of that layer. Furthermore, the number of layers 176 defining the matrix may be such that portions of saliva 140 may be anticipated to change direction of movement a number of times while passing through carrier 170, resulting in the non-smooth flow of the saliva through the carrier. It is hypothesized by the inventors that such a non-smooth flow of saliva 140 through carrier 170 may facilitate dissolving surfactant 172 into the saliva.

For some applications, and as shown, once system 100 is in the state shown in Fig. 2C, carrier 170 has also become compressed (e.g., between sponge 130 and outlet 168), thereby facilitating movement of saliva 140 and surfactant 172 out of the carrier and through outlet 168. That is, for some applications, the dimensions of structure 120 (e.g., plunger 122 thereof) and compressibility of carrier 170 are such that sliding of distal portion 124 of the plunger through channel 164 compresses the carrier between sponge 130 and outlet 168.

For some applications, sponge 130 is more compressible than carrier 170, e.g., such that, before the carrier becomes maximally compressed, the carrier receives at least a portion (e.g., the majority of) of saliva 140 that will be extracted from the sponge.

For some applications, and as shown in Fig. 2D, collection tube 180 may be decoupled (e.g., unscrewed) from adapter 182 when a desired volume of saliva 140 and surfactant 172 (i.e., surfactant 172 dissolved into the saliva) is received in the collection tube. For some applications, the desired volume may be between 100 and 1000 microliters (e.g., between 300 and 600 microliters). For some such applications, and as shown in Fig. 2D, a portion of the saliva and/or a portion of surfactant 172 may remain in carrier 170 when the desired volume is received in the collection tube 180.

For some applications, upon decoupling collection tube 180 from adapter 182, the adapter automatically seals (e.g., due to closing of a valve (not shown) disposed in the adapter). That is, upon decoupling collection tube 180 from adapter 182, the adapter inhibits release of saliva 140. For some such applications, upon decoupling collection tube 180 from adapter 182, the adapter inhibits recoupling of collection tube 180 to the adapter.

Typically, and as shown in Fig. 2E, collection tube 180 is sealed. For some applications, and as shown, collection tube 180 is sealed by closing a cap 196 thereof. For some applications, collection tube 180 is sealed automatically (i.e., the tube is a "self-sealing" tube) upon being decoupled from outlet 168.

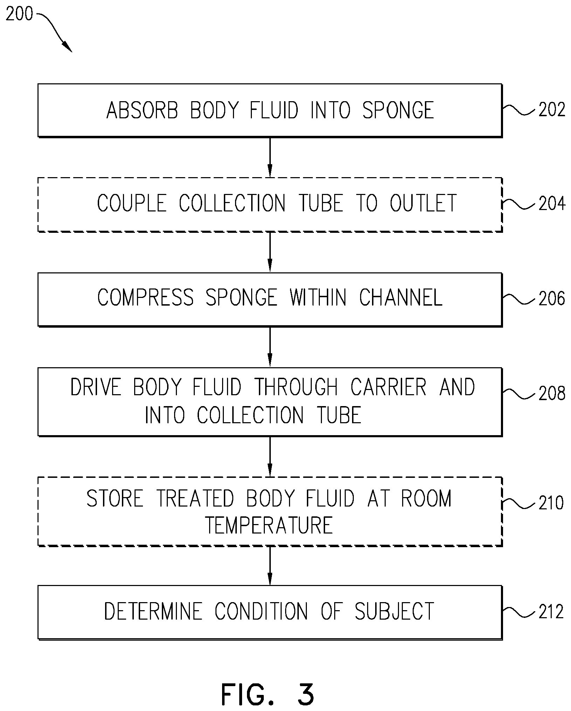

Reference is made to Fig. 3, which is a flowchart that schematically illustrates at least some steps of a method 200 that is performed in accordance with some applications of the invention. Typically, method 200 is performed using system 100 and/or techniques described hereinabove.

Saliva 140 is absorbed into sponge 130 (step 202), such that the saliva is held by the sponge, as described hereinabove. For some applications, sponge 130 is bathed in saliva 140. Typically, distal portion 124 of plunger 122 is placed in the mouth of the subject, where sponge 130 absorbs the saliva. For some applications in which sponge 130 comprises an indicator that indicates that the sponge holds a desired volume of saliva 140, the sponge is bathed in the saliva until the indicator indicates that the sponge holds the desired volume of saliva.

Typically for applications in which system 100 is provided without collection tube 180 being coupled to outlet 168 of barrel 162 (e.g., applications in which collection tube 180 is provided separately from structures 120, 160) the collection tube is then coupled to the outlet (optional step 204).

Plunger 122 is then advanced distally within barrel 162, compressing sponge 130 within channel 164 (step 206). For some applications, plunger 122 is used to press sponge 130 against carrier 170. For some applications, sponge 130 is compressed by screwing plunger along screw thread 161 of structure 160.

Compression of sponge 130 within channel 164 typically drives saliva 140 through carrier 170 and into collection tube 180 (step 208). Typically for such applications, saliva 140 mixes with and/or dissolves surfactant 172 while passing through carrier 170. In this way, saliva 140 and surfactant 172 typically exit outlet 168 and enter collection tube 180. For some applications, compression of sponge 130 may be halted (e.g., when a desired volume of saliva 140 is received in collection tube 180), regardless of whether a portion of the saliva and/or surfactant 172 remains in carrier 170.

For some applications, saliva 140 is then stored at room temperature, e.g., within collection tube 180, (optional step 210) until the saliva is transferred for use in a diagnostic assay to determine a condition of the subject (step 212). Among diagnostic assays that may be performed are Polymerase chain reaction (PCR) assay, Fluorescence-activated cell sorting (FACS), Enzyme-linked immunosorbent assay (ELISA), or Immunochromatographic assays. This list is not meant to be exclusive of other diagnostic assay techniques.

Saliva 140 processed according to steps 202, 206 and 208 may typically be stored at room temperature for at least one hour (e.g., for up to 72 hours). It is hypothesized by the inventors that storing the saliva at room temperature, prior to use in a diagnostic assay, provides certain advantages over other methods of treating saliva for diagnostic purposes (e.g., over methods that require immediate use of the saliva in a diagnostic assay, or those that require refrigeration and/or freezing of the saliva during the hours that pass from collection of the saliva until use of the saliva in a diagnostic assay). For example, processing saliva 140 as described hereinabove may facilitate collection and processing of the saliva by untrained users (e.g., self-collection by a patient) at home or at point-of-care. Furthermore, obviation of refrigeration or freezing during storage of saliva samples may (i) reduce costs of transportation and storage, (ii) enable economies of scale when performing the diagnostic assays, since samples may be stored until larger batches of samples are available for testing in parallel, and (iii) enable greater reproducibility of results, since more saliva samples may be tested simultaneously under standardized conditions.

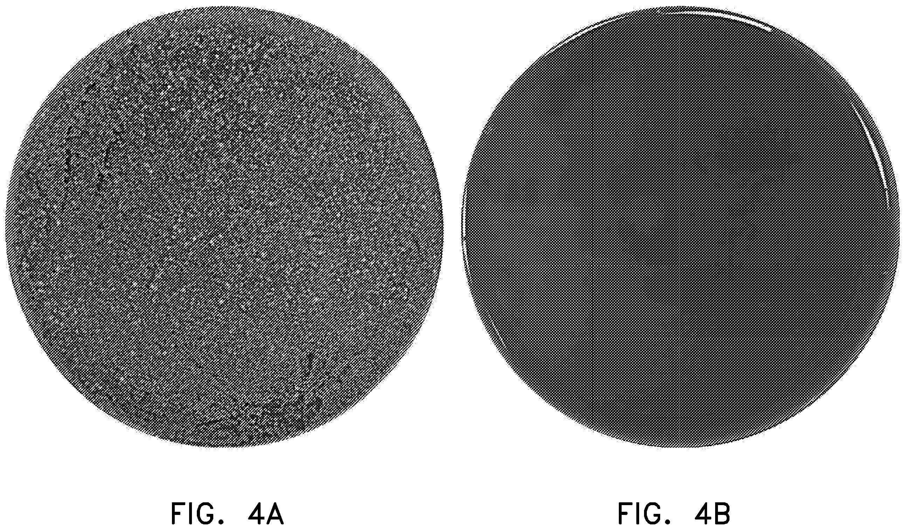

Reference is made to Figs. 4A-B, which show results of an experiment performed on untreated saliva, or on saliva treated according to an application of the disclosure. Samples of saliva that were either treated according to method 200 described hereinabove (Fig. 4B), or untreated (Fig. 4A) were incubated in agar plates under standard conditions for 72 hours. As seen in Fig. 4A, bacteria and/or fungi grew abundantly in the agar plate with untreated saliva, with over 90 percent confluence. In contrast, the agar plate with saliva treated according to method 200 shows no evidence of bacterial or fungal growth (Fig. 4B). It is hypothesized by the inventors that prevention of bacterial and fungal growth in saliva samples stored for 72 hours may facilitate the use of those saliva samples in a variety of diagnostic assays (e.g., by preserving the structural integrity of molecular substrates to be assayed).

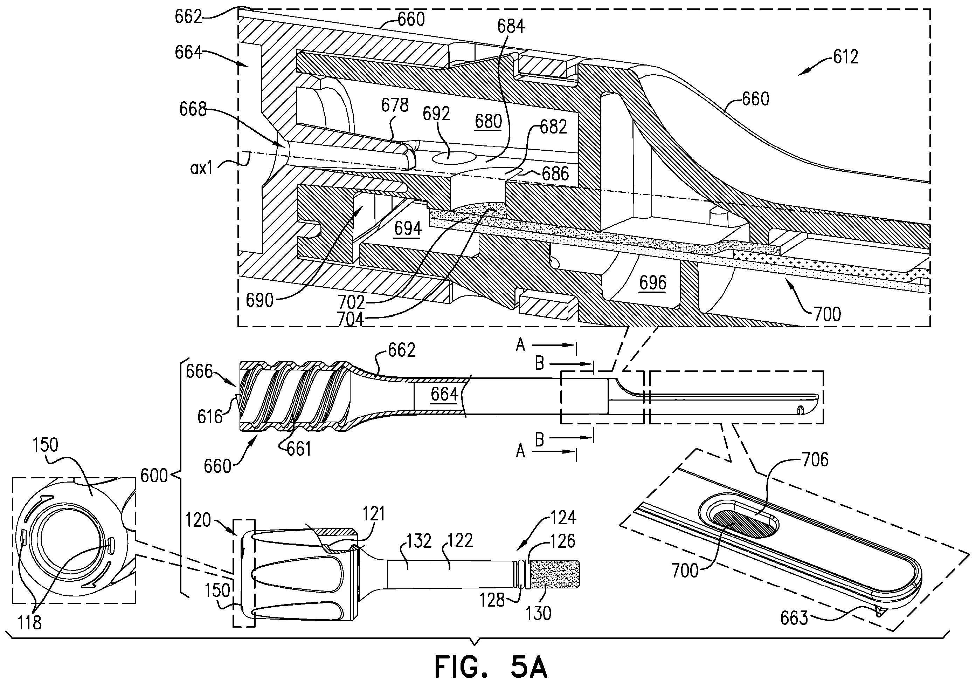

Reference is now made to Figs. 5A-B, 6, 7 and 8, which are schematic illustrations of a system 600 for use with saliva 140 of a subject, in accordance with some applications of the invention.

System 600 is in some ways similar to system 100 described hereinabove. For example, system 600 comprises first structure 120 (e.g., a sampler), having plunger 122 that is coupled to a sponge 130, as described hereinabove.

Similarly to second structure 160 of system 100, system 600 has a casing 660 that defines a channel 664 therethrough. As shown in Fig. 5A, channel 664 has an opening 666 into channel 664 at a proximal region of the channel, and an outlet 668 from the channel at a distal region of the channel. As described hereinabove with reference to system 100, plunger 122 is dimensioned to slide snugly distally within channel 664. Thus, when sponge 130 of sampler 120 holds a sample of saliva, advancing plunger 122 within channel 664 via opening 666 compresses the sponge and pushes the sample out of the sponge and through outlet 668.

For some applications, and as shown in Fig. 5A, system 600 comprises a lock, e.g., comprising a first locking element 616 (such as a tooth) and a second locking element 118 (such as a notch in a base 150 of structure 160) shaped and positioned to mate with each other upon sampler 120 being fully inserted into casing 660. Typically, the lock is configured to lock effectively irreversibly (i.e., irreversibly under normal use, without use of excessive force). It is hypothesized by the inventors that the lock further increases the efficacy and hygiene of system 600.

For some applications, and similarly to system 100, channel 664 houses a porous carrier (such as carrier 170 described hereinabove) that is disposed proximally from outlet 668. For some such applications, the carrier holds one or more dry buffer components (e.g., surfactant). Therefore, compression of sponge 130 that holds saliva within channel 664 pushes the saliva (i) out of the sponge and into the carrier, dissolving at least some of the buffer components, and (ii) out of the carrier with the dissolved buffer components, through outlet 668.

However, in contrast to system 100 that is used to drive saliva into collection tube 180, casing 660 houses a lateral flow platform 700 (e.g., sample pad 702 thereof) that is in fluid communication with outlet 668. Lateral flow platform 700 typically has a sample pad 702 for detection of an analyte, as described hereinbelow.

For some applications, channel 664 contains a porous carrier and a stationary phase (such as a cellulosic stationary phase) disposed proximally from outlet 668 and/or disposed distally from the carrier. For some applications in which channel 664 contains both a stationary phase and a porous carrier, compression of sponge 130 holding saliva 140 by sliding distal portion 124 through channel 664 drives the saliva (i) out of the sponge, (ii) through the porous carrier (iii) with the dissolved buffer components, into the stationary phase, and (iv) as an eluate that is eluted from the saliva, out of the stationary phase and through outlet 668. For some such applications, the porous carrier holds one or more dry buffer components, and the saliva dissolves at least some of the buffer components as the saliva is driven through the carrier. Typically for such applications, at least some of the dissolved buffer components are present in the eluate that is eluted from the saliva.

For some applications, channel 664 contains a stationary phase, but does not contain a carrier. Typically for such applications, compression of sponge 130 holding saliva 140 by sliding distal portion 124 through channel 664 drives the saliva (i) out of the sponge, (ii) into the stationary phase, and (iii) as an eluate that is eluted from the saliva, out of the stationary phase and through outlet 668.

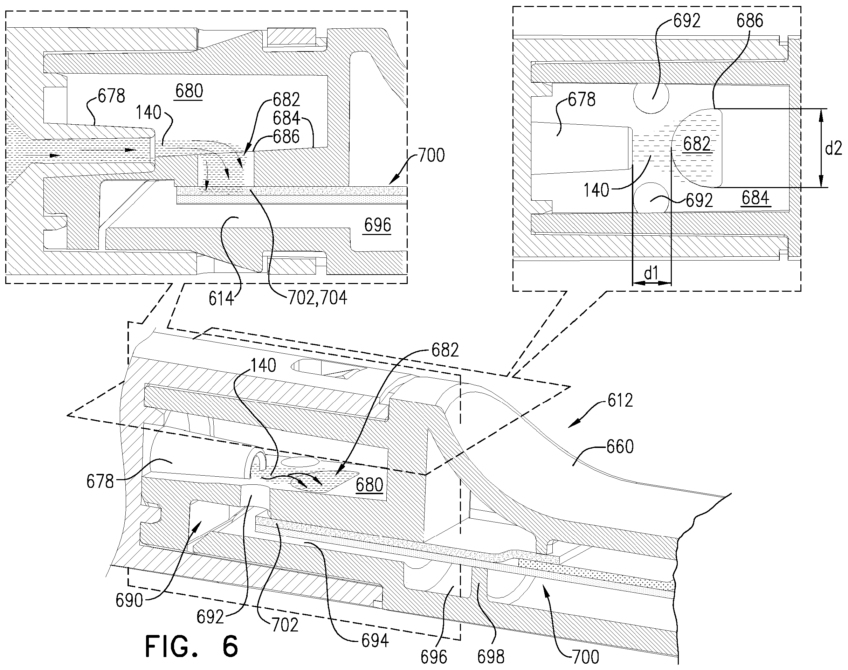

System 600 is in some ways similar to system 100 described in US Patent Application Publication 2020/0386752 to Deutsch et al. (e.g., with reference to Figs. 3 and 4A-B thereof. For example, casing 660 is in some ways similar to structure 160 described in US Patent Application Publication 2020/0386752 to Deutsch et al. System 600 and/or techniques for use thereof may be therefore used in combination with those described in reference to Figs. 3 and 4A-B of US Patent Application Publication 2020/0386752 to Deutsch et al. In contrast to system 100 described in US Patent Application Publication 2020/0386752 to Deutsch et al., system 600 comprises a flow-regulation component 612, and sampler 120 for collecting a sample of the saliva and introducing the saliva into the tester. As shown in Fig. 5A, casing 660 comprises (or is shaped to define) a chamber 680, and a nozzle 678 that extends from outlet 668 and opens into the chamber. Chamber 680 has a floor 684 that is shaped to define an aperture 686 therethrough. At least a portion 704 of sample pad 702 is disposed below aperture 686, such that the aperture and the portion of the sample pad collectively define a pool 682, the aperture defining a rim of the pool, and the portion of the sample pad defining a bottom of the pool. In this way, nozzle 678 directs the sample from outlet 668 and toward the rim of pool 682. As shown in Fig. 6, width d2 represents the width of aperture 686 (and therefore pool 682).

Typically, the user of system 600 is the subject whose saliva is being tested. That is, system 600 is typically a self-testing system. Therefore, flow-regulation component 612 is configured to optimize flow of saliva 140 and/or an eluate eluted therefrom from outlet 668 to sample pad 702 of lateral flow platform 700.

System 600 is typically configured for horizontal use. At least after distal portion 124 of sampler 120 has been inserted into channel 664 of casing 660, the system is typically placed on a flat surface while testing takes place. System 600 (e.g., casing 660 thereof) may comprise at least one leg 663 (Fig. 5A), shaped to stabilize system 600 on the surface, e.g., to inhibit the system from rolling.

Casing 660 typically defines a viewing window 706 via which the user may view the results of the test, e.g., similarly to existing commercial pregnancy tests. Therefore, system 600 may be considered to have a top side (at which window 706 is disposed) and a bottom side (at which leg 663 is disposed). It is to be noted that throughout this patent application (including the specification and the claims), the terms "above" and "below" (or similar terms, such as "upward" or "downward") refer to relative positions in this orientation.

For some applications, leg 663 is dimensioned such that, when system 600 is resting on a flat surface, at least lateral flow platform 700 slopes distally downward at a shallow angle (e.g., at 1-5 degrees, e.g., at 2-4 degrees, such as 3 degrees) with respect to the surface. For some such applications, nozzle axis ax1, and/or system 600 as a whole, slopes distally downward in this manner.

System 600 is configured for easy use, such as by an untrained individual, e.g., a consumer. When testing a sample of saliva 140 using a lateral flow assay, it is typically important to control the volume of the saliva introduced to the assay - e.g., to ensure that a specific volume of the saliva is introduced to the assay. For example, too small a volume may result in insufficient flow (e.g., sufficient quantities of the target analyte may not reach the antibodies of the assay), while too large a volume may result in uncontrolled and/or nonlinear flow of the sample through and/or out of the sample pad or other pads/beds/membranes of the lateral flow platform, thereby reducing sensitivity of the assay. Sample volume control is typically facilitated at least in part by sponge 130 having a known capacity, and is further facilitated by the dimensions of channel 664 (and optionally barrel 662) and sampler 120 providing controlled compression of the sponge.

Sample volume control, and therefore sensitivity of the assay, is further facilitated by features of flow-regulation component 612 that facilitate controlled transfer of saliva 140 from outlet 668 of channel 664 to sample pad 702 of lateral flow platform 700. In order to test the contribution of flow-regulation component 612 to the diagnostic sensitivity of system 600, the inventors performed a saliva HCG-detection experiment comparing the performance of system 600 to that of system 100 described with reference to Figs. 3 and 4A-B of US Patent Application Publication 2020/0386752 to Deutsch et al.

In the experiment, either low volumes (300-400 microliters) or high volumes (400-750 microliters) of saliva were absorbed into sponge 130. When detecting HCG in low volumes of saliva, both systems 100 and 600 had generally identical sensitivity of approximately 1 milli-international units per milliliter.

However, when detecting HCG in high volumes of saliva, system 100 demonstrated decreased sensitivity, resulting from a sample volume that is higher than the optimal capacity of the sample pad of lateral flow platform 700 (e.g., resulting in non-linear flow of the sample through the sample pad). In these cases, decreased assay sensitivity was observed, due to an increased number of false-negatives (sometimes to the point where there was no detectable signal). Sensitivity of system 600 remained, by contrast, approximately 1 milli-international units per milliliter.

It is hypothesized by the inventors that the superior sensitivity demonstrated by system 600 for detection of HCG in the high-volume saliva samples may be attributed to flow-regulation component 612, which prevents excessively large volumes of saliva from reaching sample pad 702 of lateral flow platform 700.

Features of flow-regulation component 612 include a nozzle 678, a chamber 680, and a pool 682, and may also include an overflow system 690, which are described in detail hereinbelow. It is hypothesized by the inventors that these features provide system 600 with additional tolerance regarding sample volume. For example, sponge 130 may be configured to have a capacity that is slightly higher than the volume of saliva 140 that would ideally be introduced to sample pad 702, allowing for situations in which the user inadvertently does not completely fill the sponge with saliva. For some such applications, sponge 130 has a capacity of 100-800 microliters (e.g., 300-800 microliters, e.g., 400-600 microliters, e.g., 500-600 microliters, such as about 550 microliters) of liquid (e.g., of saliva).

For some applications, pool 682 and chamber 680 are dimensioned to regulate (e.g., to limit) the flow of saliva 140 from nozzle 678 to sample pad 702. For some such applications, pool 682 has a volume that is smaller than the capacity of the sponge. For some such applications, chamber 680 has a volume (separate from the volume of pool 682) that is greater than the capacity of the sponge.

As shown, nozzle 678 opens into the chamber in an orientation that directs saliva 140 from outlet 668 toward the rim of pool 682. For some applications, and as shown, channel 664 is collinear with nozzle 678.

The orientation of nozzle 678 defines a longitudinal nozzle axis ax1, and sample pad 702 is typically disposed below the nozzle axis (i.e., below a nozzle plane coincident with the nozzle axis). Typically, and as shown, most of chamber 680 (i.e., most of the hollow volume of the chamber) is disposed above nozzle axis ax1 (e.g., above the nozzle plane).

For some applications, and as shown, sample pad 702 is parallel with nozzle axis ax1 (e.g., parallel with the nozzle plane). Fig. 6 shows saliva 140 flowing from nozzle 678 toward the rim of pool 682, and over the rim into the pool, from where it soaks into sample pad 702.

It is to be noted that system 600 is configured such that, although saliva 140 is forced (i.e., under pressure delivered by plunger 622) through outlet 668 toward lateral flow platform 700, the saliva is not forced into or along the lateral flow platform (e.g., into or along the sample pad or the other pads/beds/membranes of the lateral flow platform). Rather, once saliva 140 reaches chamber 680, the saliva flows into pool 682 and soaks into sample pad 702, from where the saliva passes through the lateral flow platform via capillary action. Typically, this is achieved at least in part by chamber 680 being sufficiently large to contain saliva 140, and/or the chamber being not hermetically sealed, therefore allowing elevated pressure to be released to outside of the chamber (e.g., to outside of casing 660) as the saliva enters the chamber, e.g., as the saliva displaces air within the chamber.

Typically, and as shown, sample pad 702 (or at least portion 704 thereof) is disposed tightly against the underside of floor 684, such that saliva 140 in pool 682 may proceed only after soaking into the sample pad. For example, the tight placement typically prevents saliva 140 from flowing distally over the upper surface of sample pad 702.

For some applications, system 600 (e.g., casing 660) provides an overflow system 690 that comprises one or more overflow holes 692 and a spillway 694. Overflow system 690 is configured to direct excess saliva out of the chamber via the one or more overflow holes to the spillway, e.g., in order to prevent too large a volume of saliva entering lateral flow platform 700. Typically, overflow holes 692 are defined by floor 684, and lead through the floor to spillway 694. Typically, and as shown, at least part of spillway 694 is disposed below floor 684. Furthermore, for some applications, and as shown, at least part of spillway 694 is disposed below at least part of lateral flow platform 700, e.g., below sample pad 702. For some applications, and as shown spillway 694 may define a widened portion (e.g., a reservoir) 696 (e.g., at a distal end of the spillway), to store the excess saliva.

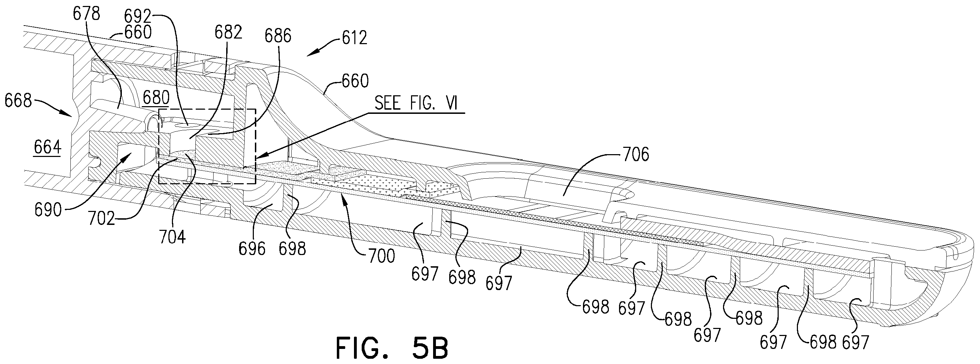

For some applications, and as shown in Fig. 5B, casing 660 defines one or more cavities 697 distal to portion 696, arranged in series longitudinally, disposed below lateral flow platform 700, and separated from each other by baffles 698 defined by the casing. Baffles 698 are configured to allow limited fluid communication between cavities 697, such that excess saliva 140 may move distally from portion 696, but by separating the overall volume below lateral flow platform 700 the baffles obstruct bulk flow or "sloshing" of saliva 140 that may have entered this part of casing 660.

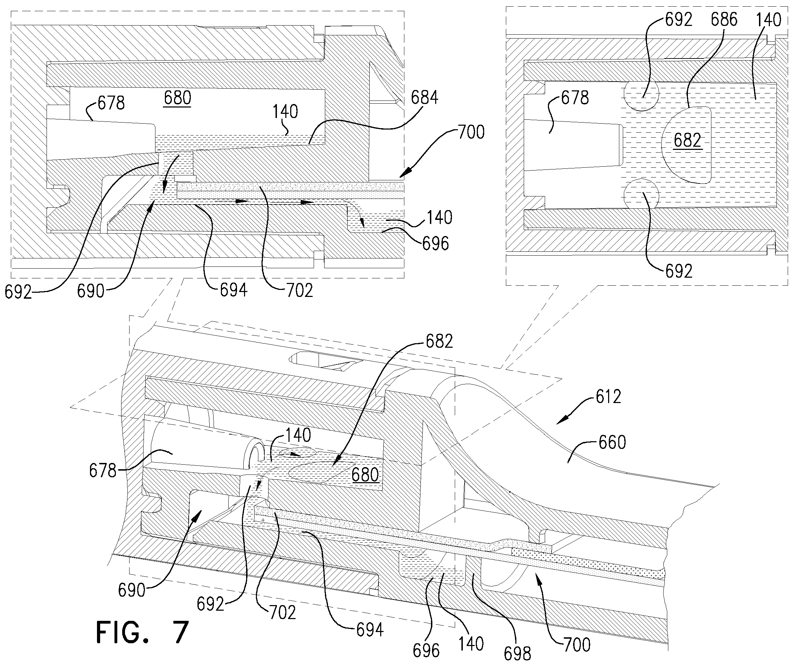

For some applications, and as shown, overflow holes 692 are disposed proximally from the aperture. This positioning is hypothesized by the inventors to facilitate optimization of the volume of saliva 140 that enters lateral flow platform 700. Fig. 7 shows an undesirably large volume of saliva 140 having been introduced into chamber 680, and excess of the saliva flowing through overflow holes 692 into spillway 694.

For some applications, and as shown in Figs. 6-7, overflow system 690 comprises a first overflow hole 692 and a second overflow hole 692, that are disposed on either side of a direct path between the nozzle and the aperture, e.g., as shown. This positioning of overflow holes 692 is hypothesized by the inventors to facilitate optimization of the volume of saliva 140 that enters lateral flow platform 700. This positioning is hypothesized by the inventors to, for some applications, provide an additional advantage of facilitating the directing of saliva 140 distally from nozzle 678 toward the rim of pool 682, by inhibiting the saliva from dispersing laterally, e.g., due to surface phenomena such as surface tension.

For some applications, in addition to directing the saliva distally from the nozzle toward the rim of the pool, and channeling away excess saliva, overflow holes 692 also allow bubbles that may form in saliva to exit chamber 680, and/or facilitate the above-described pressure release from the chamber.

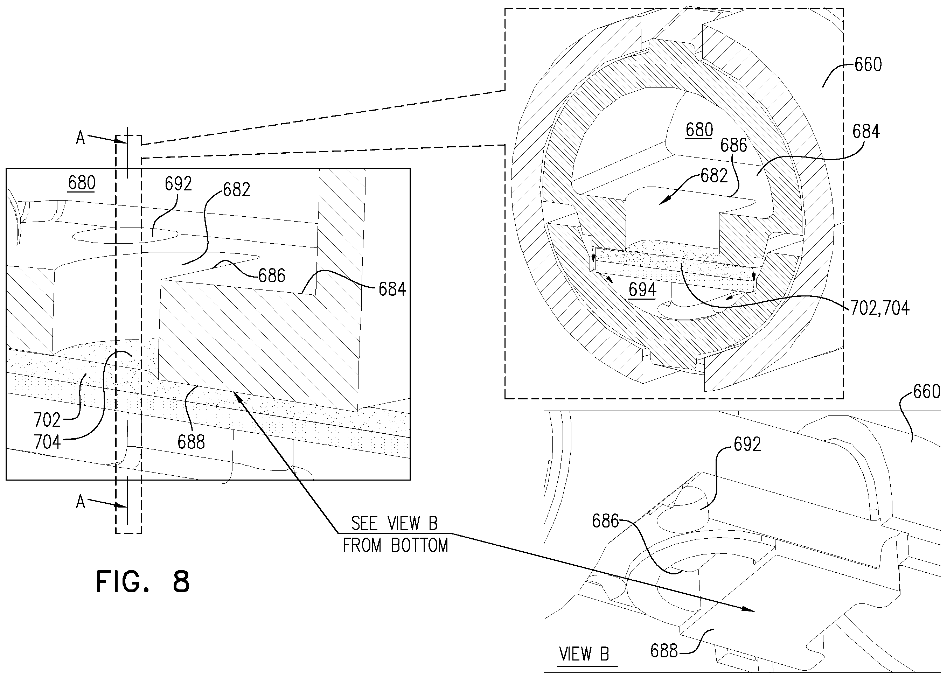

For some applications, and as shown in Fig. 8, the underside of floor 684 is shaped such that pressing of the floor against sample pad 702 is different at different positions around the bottom of pool 682. For such applications, this pressing is typically greater at the distal side of pool 682 than at the proximal side or lateral sides of the pool. As shown in Fig. 8, this may be achieved by a region 688 of the underside of floor 684 protruding downward further (and therefore pressing against sample pad 702 more) than more proximal portions of the underside of the floor. It is hypothesized by the inventors that this configuration advantageously further allows excess saliva 140 and/or pressure to escape into spillway 694, by flowing laterally (and possibly proximally) outward from the bottom of pool 682 (i.e., between sample pad 702 and the underside of floor 684, as indicated by the arrows in Fig. 7), while inhibiting the saliva from flowing distally over the upper surface of the sample pad.

For some applications, and as shown, floor 684 slopes distally upward - i.e., is sloped such that progressively distal parts of the floor are progressively higher (e.g., irrespective of whether sample pad 702 is parallel with nozzle axis ax1). For some such applications, a proximal part of floor 684 is disposed below nozzle axis ax1 (e.g., below the nozzle plane), and the floor slopes distally upward to intersect the nozzle axis (e.g., to intersect the nozzle plane). For example, the floor may slope distally upward at more than 1 degree (e.g., more than 2 degrees) and/or less than 10 degrees (e.g., less than 6 degrees, e.g., less than 4 degrees) with respect to the nozzle axis, e.g., 1-10 degrees (e.g., 1-5 degrees, e.g., 2-4 degrees, such as 3 degrees) with respect to the nozzle axis. It is hypothesized by the inventors that, for some applications, this sloping further facilitates optimization of the volume of saliva 140 that enters lateral flow platform 700, e.g., by encouraging excess saliva 140 to flow away from pool 682 and/or toward overflow holes 692.

For some applications in which (i) leg 663 is dimensioned such that nozzle axis ax1 slopes distally downward at a shallow angle with respect to the surface on which system 600 is resting, and (ii) floor 684 slopes distally upward with respect to the nozzle axis, these slopes are similar in magnitude, such that the floor is parallel with the surface on which the system is resting.

For some applications, the analyte that system 600 (e.g., lateral flow platform 700 thereof) is configured to detect comprises human chorionic gonadotropin (HGC). For example, the presence and/or concentration of HCG may be detected, and it is determined that the subject is pregnant or not pregnant, based upon the detected presence and/or concentration of HCG. Alternatively or additionally, a stage of a subject's pregnancy may be determined based upon the determined presence and/or concentration of HCG.

For some applications, the analyte that system 600 is configured to detect comprises luteinizing hormone (LH). For example, the presence and/or concentration of LH may be detected, and based upon the detected presence and/or concentration of LH, a stage in the menstrual cycle of the subject may be determined - e.g., it may be determined whether the subject is currently ovulating.

For some applications, the analyte that system 600 is configured to detect comprises a component of a virus, such as a virus that is present in the mouth or nose of an individual during infection with the virus (e.g., a virus that infects the respiratory system or the gastrointestinal system). For example, the analyte may comprise a component of a coronavirus (e.g., a SARS-related coronavirus, such as SARS-CoV-2, the cause of COVID-19) or an orthomyxovirus (e.g., an influenza A virus or an influenza B virus). For example, system 600 may be used to screen passengers at mass transit terminals such as ports, airports, train stations, and bus stations, e.g., during an outbreak of a viral disease, in order to inhibit the spread of the disease.

For some applications, the analyte that system 600 is configured to detect comprises histidine-rich protein (HRP) II and/or pan-Plasmodium lactate dehydrogenase antigen. For example, the presence and/or concentration of histidine-rich protein 2 and/or pan-Plasmodium antigen lactate dehydrogenase is detected, and it is determined that the subject is suffering from malaria, or is not suffering from malaria, based upon the detected presence and/or concentration of histidine-rich protein 2 and/or pan-Plasmodium antigen lactate dehydrogenase.

For some applications, the analyte that system 600 is configured to detect comprises a Helicobacter pylori (H. pylori) antigen, such as CagA. For example, the presence and/or concentration of the H. pylori antigen is detected, and it is determined that the subject carries H. pylori, or does not carry H. pylori, based upon the detected presence and/or concentration of the H. pylori antigen.

For some applications, the analyte that system 600 is configured to detect comprises a Candida spp. (e.g., C. albicans) antigen, such as Candida albicans enolase. For example, the presence and/or concentration of the Candida antigen is detected, and it is determined that the subject carries Candida, or does not carry Candida, based upon the detected presence and/or concentration of the Candida antigen.

For some applications, the analyte that system 600 is configured to detect comprises a medical or recreational drug, a component thereof, and/or a metabolite thereof. For example, the presence and/or concentration of tetrahydrocannabinol (THC) is detected, and it is determined that the subject has consumed cannabis or a cannabis product, based upon the detected presence and/or concentration of THC. Alternatively or additionally, a concentration of the drug, drug component, or component thereof within the subject (e.g., within the blood of the subject) may be determined based upon the detected presence and/or concentration of the drug, drug component, or component thereof. Alternatively or additionally, the time since consumption of the drug may be determined based upon the detected presence and/or concentration of the drug, drug component, or component thereof. For applications in which the drug is a medical drug, dosing of the medical drug may also be controlled and/or adjusted based on the detected presence and/or concentration of the drug, drug component, or component thereof.

For some applications, the analyte that system 600 is configured to detect comprises troponin. For example, the presence and/or concentration of troponin is detected, and it is determined that the subject has experienced a myocardial infarction, based on the detected presence and/or concentration of troponin.

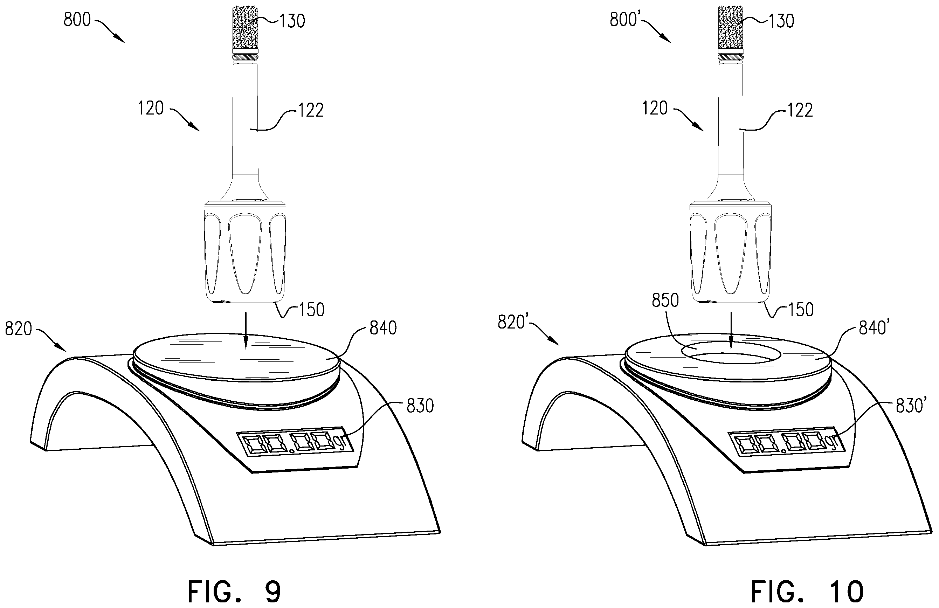

Reference is made to Figs. 9-10, which are schematic illustrations of systems 800, 800' for use with saliva of a subject, in accordance with some applications of the disclosure.

As described hereinabove, sampler 120 is used to collect a sample of saliva from the mouth of a subject. It may desirable to determine whether at least a predetermined amount (e.g., the predetermined amount being in the range 0.2 to 1.0 g, such as 0.5 g) of saliva has been collected into sponge 130. Systems 800, 800' therefore comprise a scale 820, 820' for weighing sampler 120. Sampler 120 may be weighed prior to collecting the saliva, or the sampler may have a known weight that may be subtracted from the measured weight of a sampler that holds saliva. In either case, sampler 120 is typically placed on a platform 840, 840' of scale 820, 820'.

For some applications, and as shown, the weight of sampler 120 is displayed on display 830, 830' of scale 820, 820'. Alternatively or in addition, scale 820, 820' may indicate by other means (e.g., a visual or an auditory signal) whether the weight of sampler 120 demonstrates that the sampler holds at least the predetermined amount of saliva. It is hypothesized by the inventors that regulating the collected saliva volume by weighing sampler 120 prior to using the sampler in a diagnostic assay (e.g., with systems 600 or 900) may improve (i) assay performance (e.g., by preventing a reduction in signal intensity and/or result quality, such as color-smearing effects, caused by low sample volume), and (ii) analytical performance (e.g., by preventing a longer than optimal incubation time during which an insufficient volume of sample may incubate on the sample pad).

For some applications, and as shown in Fig. 10, platform 840' has a receiver 850' that is configured to facilitate coupling sampler 120 to the platform and/or stabilizing the sampler on the platform. For example, and as shown, receiver 850' may take the form of a depression in platform 840' that is dimensioned to fit base 150 of sampler 120. Description of receiver 850' as a depression is not meant to be limiting, and is not meant to exclude other embodiments in which the receiver may be shaped differently (e.g., as a projection), or may operate based on a different principle (e.g., by magnetic coupling of base 150 to platform 840').

Coupling sampler 120 to scale 820' reduces a risk of the sampler falling from the scale or otherwise becoming contaminated. In the case that sampler 120 is determined to not have the predetermined amount of saliva, the sampler may then be used to recollect saliva from the subject, without a risk of contamination.

After it is determined that sponge 130 holds at least the predetermined amount of saliva, sampler 120 is typically used in a diagnostic procedure, e.g., involving other components of systems 100 or 600 described hereinabove, or system 900 described hereinbelow.

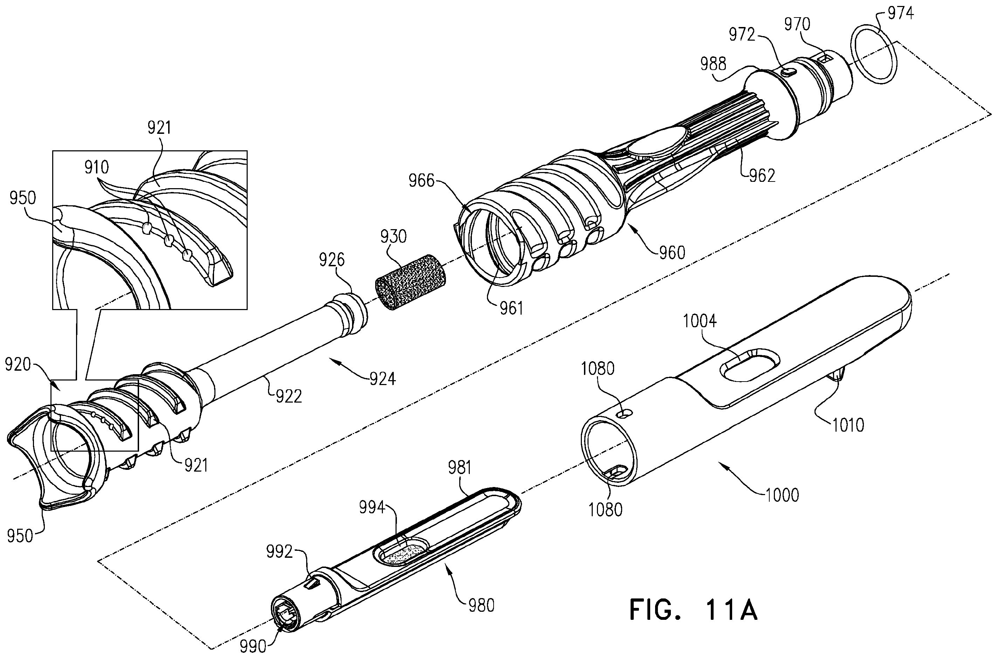

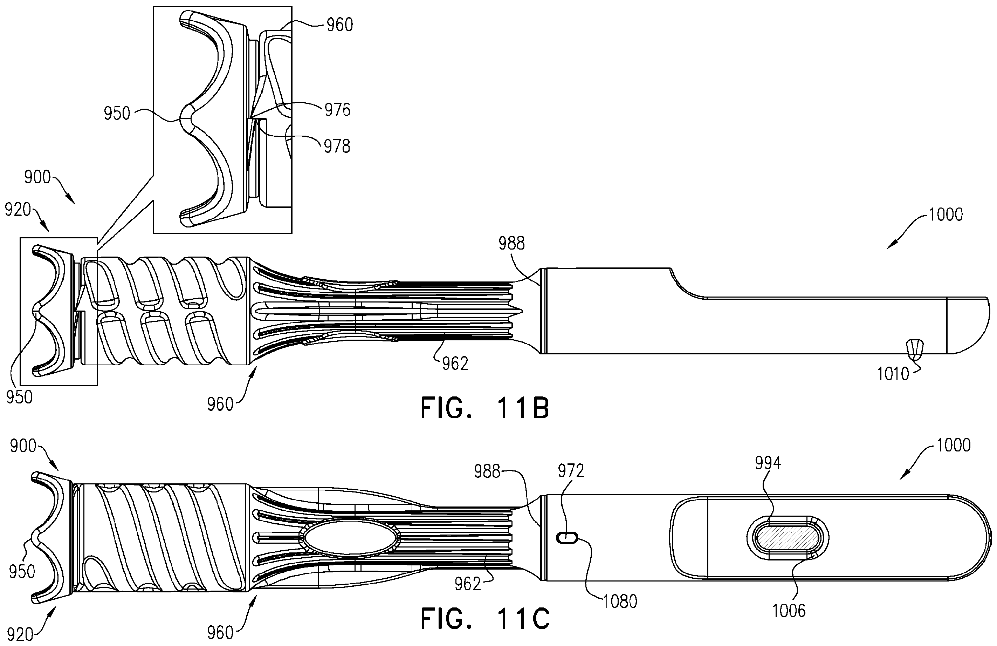

Reference is made to Figs. 11A-C, 12 and 13A-C, which are schematic illustrations showing a system 900 for use with saliva of a subject, in accordance with some applications of the disclosure.

System 900 is in some ways similar to system 600 described hereinabove. Components that are identically named between the systems typically share similar features and serve similar functions as each other. As such, the description below of system 310 focuses upon features that are particular to system 310.

As shown in the exploded view of system 900 shown in Fig. 11A, a sponge 930 is couplable to a distal portion 924 of a plunger 922 (e.g., of a sampler 920). The sponge is configured to hold saliva, and is typically introduced, using plunger 922, via an opening 966 at a proximal region of a barrel 962 (e.g., of a receptacle 960), into a channel that is defined by the barrel. As described hereinabove with reference to system 100, a porous carrier is disposed within the channel, between an upstream opening 966 and a downstream outlet.

Similarly to as in system 600, sponge 930 is dimensioned to slide snugly within the channel, such that sliding of distal portion 924 through the channel compresses the sponge within the channel. Therefore, while the sponge holds the saliva, compression of the sponge within the channel drives the saliva out of the sponge. Since system 600 typically comprises a porous carrier within the channel, compression of the sponge within the channel further drives the saliva through the carrier. The saliva then (i) exits barrel 962 through an outlet at a distal region of the barrel, and (ii) enters a lateral flow platform 980 that is coupled to the distal region of barrel 962. A sample pad of lateral flow platform 980 is typically in fluid communication with the outlet.

Similarly to systems 100 and 600, sponge 930 is compressed within the channel by helically advancing (i.e., screwing) plunger screw-thread 921 along receptacle screw-thread 961. As shown in Fig. 11A, at least one of screw-threads 921, 961 defines a protrusion 910 (e.g., a plurality of protrusions) on the respective screw-thread. For example and as shown, protrusions 910 on plunger screw-thread 921 are dimensioned such that they protrude outward from the plunger screw-thread, toward barrel screw-thread 961, as plunger 922 advances helically along the receptacle screw-thread. Protrusions 910 therefore generate greater resistance during screwing of plunger 922 along receptacle screw-thread 961, than do other portions of plunger screw-thread 921. For some such applications, this greater resistance to screwing plunger 922 may be felt by the user, and/or may be audible to the user (e.g., as a click). In this way, the user may screw plunger 922 against a first level of resistance to the screwing, and upon reaching protrusion 910, the user may briefly feel a second level of resistance and/or hear that the protrusion (e.g., the first protrusion) has engaged barrel screw-thread 961.

Protrusions 910 may therefore be disposed, on one or both of the respective screw-threads, at locations selected to aid the user in regulating the distance to which sponge 930 is advanced through the channel. That is, screwing plunger screw-thread 921 along receptacle screw-thread 961, until the portion of the plunger screw-thread that defines the protrusion 910 engages the receptacle screw-thread 961, may (i) compress sponge 930 within receptacle 960, and (ii) drive a first portion of the saliva out of the sponge, through the carrier and the outlet. Accordingly, screwing plunger screw-thread 921 along receptacle screw-thread 961, past protrusion 910, may (i) further compress sponge 930 within receptacle 960, and (ii) drive a second portion of the saliva out of the sponge, through the carrier and the outlet. For some applications, and as shown, plunger screw-thread 921 may define additional protrusions 910, past which the plunger must be screwed in order to drive an additional portion of the saliva out of the sponge, through the carrier and the outlet. In this way, protrusions 910 may aid the user to meter the amount of saliva that is driven out of the sponge, through the carrier and the outlet. That is, if the user receives an indication (e.g., a visible flow of liquid under window 994) that the saliva has reached the sample pad, after having screwed plunger 922 until the portion of the plunger screw-thread that defines the first protrusion engages receptacle screw-thread 961, the user may elect to stop screwing the plunger. If, however, the user receives no indication that the saliva has reached the sample pad, then the user may elect to continue screwing plunger 922 (e.g., until the portion of the plunger screw-thread that defines the second protrusion engages receptacle screw-thread 961). In such cases, the user may be less likely to excessively compress sponge 130 within the channel of barrel 962. Alternatively or additionally, the user may be instructed to screw plunger 922 until a given number of protrusions 910 engage receptacle screw-thread 961.

It is therefore hypothesized by the inventors that, by aiding the user to meter the amount of saliva that is driven out of the sponge and onto the sample pad of lateral flow platform 980, at least one of the screw-threads defining protrusions 910 may contribute to the sensitivity of system 900 in diagnostic assays.

For some applications, sampler 120 (e.g., base 150 thereof) and/or receptacle 960 (e.g., a proximal portion thereof) are respectively shaped to define mechanical stops 976, 978. As shown in Fig. 11B, after plunger 922 is fully advanced (e.g., screwed, as described hereinabove with reference to systems 100 and 600) into the channel of barrel 962, it may be desirable to withdraw the plunger from the barrel. Therefore, mechanical stops 976, 978 are configured to prevent withdrawal (e.g., unscrewing) of the plunger 622 from barrel 962 by offering greater resistance to withdrawal than to advancement of the plunger into the barrel.

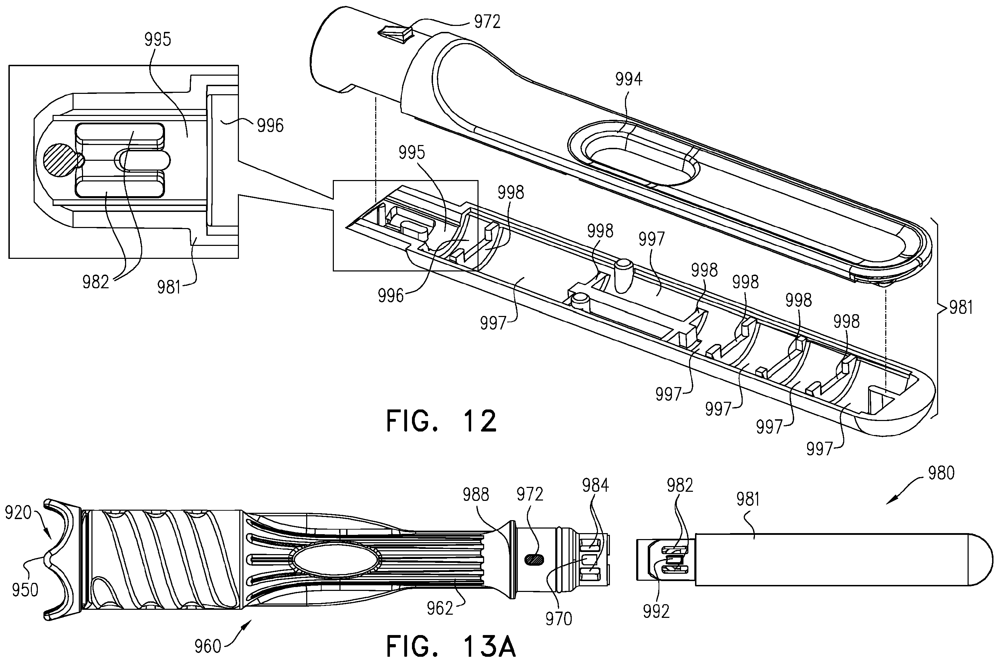

Similarly to system 600, system 900 (e.g., a casing 981 of lateral flow platform 980) provides an overflow system 990 (Fig. 11A) that is configured to direct excess saliva into a reservoir 996 and a spillway 995 (Fig. 12), e.g., in order to prevent too large a volume of saliva entering the sample pad. In contrast to casing 660, casing 981 is further shaped to define a drainage hole 982 (e.g., two drainage holes, as shown in the inset of Fig. 12). It is hypothesized by the inventors that drainage holes 982 further reduce a risk of too large a volume of saliva entering the sample pad of system 900, by draining excess saliva from within casing 981.

For some applications, and as shown, drainage holes 982 complement other features of casing 981 that obstruct bulk flow or "sloshing" of saliva through the casing. As shown in Fig. 12, casing 981 of system 900 defines cavities 997 that are disposed proximally of reservoir 996, and arranged in series longitudinally. Cavities 997 are separated from each other by baffles 998 defined by the casing. Baffles 698 are configured to allow limited fluid communication between cavities 697, such that excess saliva may move distally from reservoir 996 (e.g., from the reservoir, into spillway 995 exiting casing 981 via drainage holes 982).

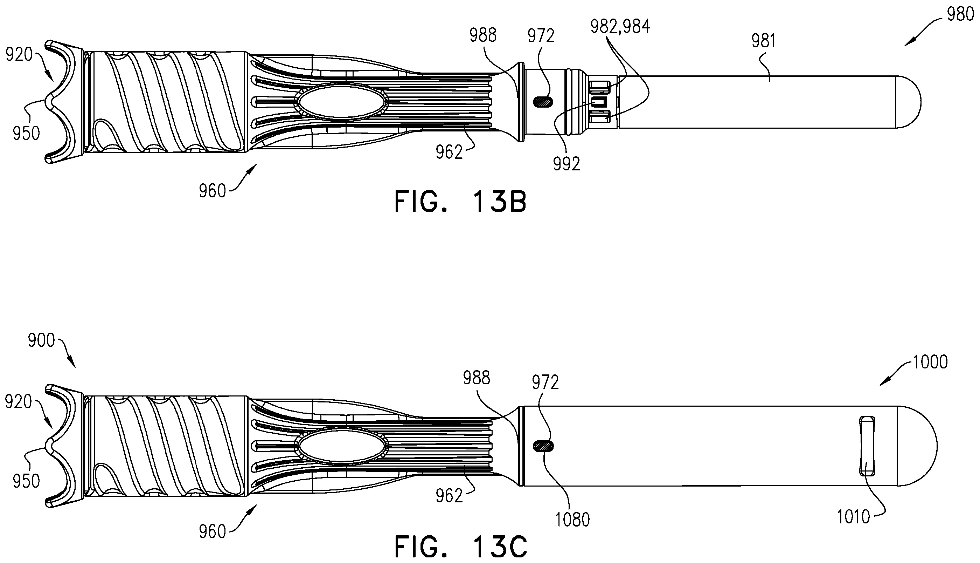

For some applications, and as shown in Figs. 13A-B, receptacle 960 (e.g., a distal portion thereof) is also shaped to define a drainage hole 984. For example, and as shown, a pair of drainage-holes 984 defined by receptacle 960 are located so as to align with a pair of drainage holes 982 defined by casing 981 (e.g., defined by a portion of the casing that is proximal of the sample pad, such as spillway 995). Therefore, when casing 981 is coupled to receptacle 960 (e.g., by casing notch 992 fitting into receptacle slot 970), excess saliva that is drained from casing 981 is also drained from receptacle 960.

It is typically not desirable for the user to come into contact with excess saliva that is drained from lateral flow platform 980. Therefore, for some applications, system 900 includes a catchment tray 1000 that is in fluid communication with drainage holes 982, 984. For some applications, and as shown, tray 1000 is coupled to receptacle 960 (e.g., such that ring 974 facilitates a leakproof seal between tray 1000 and shoulder 988 of barrel 962). In this way, when the casing is coupled to the receptacle (e.g., by barrel notch 972 fitting into tray slot 1080), excess saliva is drained from lateral flow platform 980 and collects within tray 1000.

In light of the function of drainage holes 982, 984 and tray 1000 described hereinabove, when sponge 930 is compressed within the channel of barrel 962, (i) a first portion of the saliva exits the outlet, onto the sample pad of lateral flow platform 980, and (ii) second portion of the saliva exits the outlet and the lateral flow platform, through drainage holes 982 and/or 984, and into catchment tray 1000.

For some applications, and similarly to leg 663 of casing 660 described hereinabove, tray 1000 is shaped to define at least one leg 1010 (Figs. 11A and 13C), shaped to stabilize system 600 on the surface, e.g., to inhibit the system from rolling.

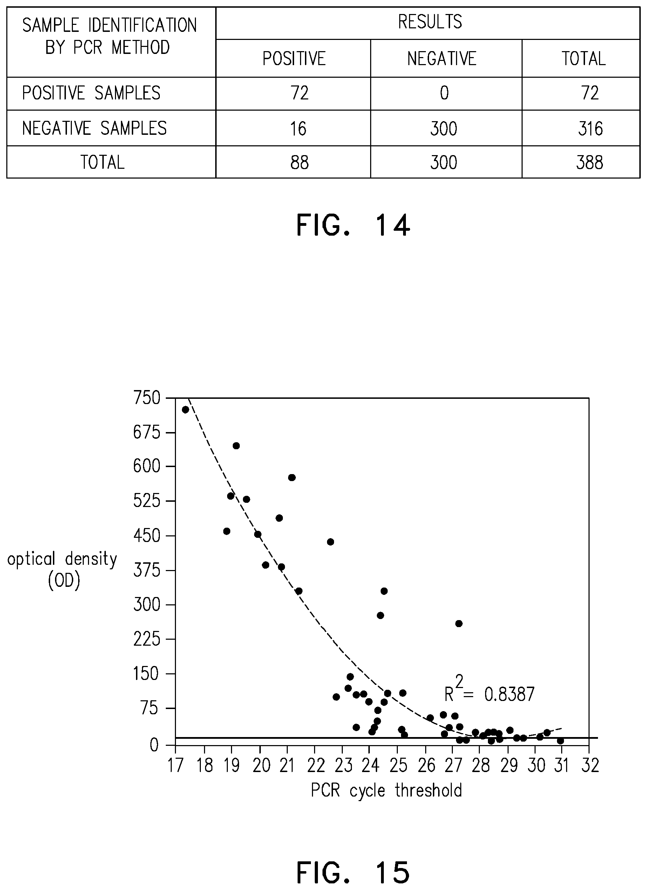

Reference is made to Figs. 14-15, which are schematic illustrations showing results of experiments conducted using system 900 for use with saliva of a subject, in accordance with some applications of the disclosure.

Fig. 14 shows the results of validation experiments performed using system 900 to detect SARS-CoV-2 in saliva samples. Real-Time Polymerase Chain Reaction (RT-PCR) testing of the same saliva samples served as a positive control, such that each saliva sample was tested using both system 900 and RT-PCR.

Saliva samples that were predetermined to be either negative or positive (88 positive samples, 300 negative samples according to RT-PCR testing of nasopharyngeal and oropharyngeal swabs from the same patients) were retrieved from a frozen sample bank and thawed. System 900 was utilized as described hereinabove, with the following modification: instead of collecting saliva directly from the subject's mouth, sponge 130 was dipped, until saturated, in 300 microliters of saliva. This modification in the saliva collection was not expected to affect the analytical performance of system 900. The results were read by researchers blind to the results of the RT-PCR testing of the nasopharyngeal and oropharyngeal swabs, according to the following endpoint criteria: (i) a visible "test" line alongside a visible "control" line was considered a positive result, and (ii) no visible "test" line alongside a visible "control" line was considered a negative result (as described with reference to Figs. 5-10 of US Patent Application Publication 2020/0386752 to Deutsch et al.). As a secondary measure, test results were also read by an automated optical density (OD) reader. The OD reader was calibrated to consider assay lines (test or control) passing an OD threshold of 17.5 as "visible," and to classify samples as positive or negative according to the same endpoint criteria as above. As for the positive control, saliva samples passing the detection threshold prior to cycle 30 (Ct<30) of RT-PCR were considered to be positive.

All 300 positive samples were detected accurately using system 900. Therefore, specificity (Negative Percent Agreement) of system 900 for detection of SARS-CoV-2 was found to be 100% (300/300 = 100% (95% CI: 98.8%-100%). Of the 88 negative samples, 72 were detected accurately using system 900. Thus, sensitivity (Positive Percent Agreement) of system 900 for detection of SARS-CoV-2 was found to be 82% (72/88 = 82% (95% CI: 72.2% - 89.2%).

Alongside the qualitative results shown in Fig. 14, the inventors compared the ability of system 900 to reflect, semi-quantitatively, the amount of SARS-CoV-2 present in the respective saliva samples. Therefore, Fig. 15 shows correlation between OD values for "test" lines in system 900 that were detected using the automated OD reader, and Ct values for the same samples in RT-PCR. The coefficient of determination R² value of 0.8387 indicates a positive correlation between the RT-PCR Ct value and the OD value attained using system 900. It is therefore hypothesized by the inventors that the OD value attained using system 900 may be used, similarly to the Ct value in RT-PCR, to reflect relative amounts of SARS-CoV-2 present in saliva samples.

It will be appreciated by persons skilled in the art that the present invention is not limited to what has been particularly shown and described hereinabove. Rather, the scope of the present invention is defined by the appended claims, and may include both combinations and subcombinations of the various features described hereinabove, as well as variations and modifications thereof that are not in the prior art, which would occur to persons skilled in the art upon reading the foregoing description.

## Claims

1. Apparatus (600), for use by a subject, the apparatus comprising:
a sampler (120), comprising:
a sponge (130), configured to be placed in a mouth of the subject, and to absorb a sample of saliva from the mouth;
a plunger (122), having a distal portion (124) that is coupled to the sponge (130);
a lateral flow platform(700), comprising a sample pad (702); and
a casing (660), housing the lateral flow platform (700), and comprising:
a channel (664), having:
an opening (666) into the channel (664) at a proximal region of the channel (664), and
an outlet (668) from the channel (664) at a distal region of the channel (664), the sampler (120) being configured to, while the sponge (130) holds the sample, introduce the sponge (130) into the channel (664) via the opening (666), the plunger (122) being dimensioned to slide snugly distally within the channel (664) in a manner that compresses the sponge (130) and pushes the sample out of the sponge (130) and through the outlet (668);
**characterized in that** the casing (660) further comprises:
a chamber (680) having a floor (684), the floor (684) defining an aperture (686) therethrough, at least a portion of the sample pad (702) disposed below the aperture (686) such that the aperture (686) and the portion of the sample pad (702) collectively define a pool (682), the aperture (686) defining a rim of the pool (682), and the portion of the sample pad (702) defining a bottom of the pool (682); and
a nozzle (678), extending from the outlet (668), and opening into the chamber (680) in an orientation that directs the sample from the outlet (668) toward the rim of the pool (682).

2. The apparatus (600) according to claim 1, wherein the casing (660) is configured to allow elevated pressure in the chamber (680), resulting from the plunger (122) sliding snugly distally within the channel (664), to be released to outside of the casing (660).

3. The apparatus (600) according to claim 1, further comprising (i) a porous carrier (170), disposed within the channel (664) proximally from the outlet (668), and (ii) one or more dry buffer components, held in the carrier (170), at least one of the buffer components being a surfactant (172) held in the carrier (170), wherein:
the plunger (122) is dimensioned to slide snugly distally within the channel in a manner that compresses the sponge and pushes the sample:
out of the sponge (130) and into the carrier (170), dissolving at least some of the buffer components, and
out of the carrier (170) with the dissolved buffer components, and through the outlet (668),
wherein the carrier (170) comprises a plurality of fibers (174) arranged such that:
spaces between the fibers (174) define pores (178) of the carrier (170), and
the fibers (174) are generally not arranged along a longitudinal axis of the carrier (170) .

4. The apparatus (600) according to any one of claims 1-3, wherein the apparatus does not comprise an albumin.

5. The apparatus (600) according to any one of claims 1-4, wherein:
the nozzle (678) defines a nozzle axis,
the channel (664) is disposed at a proximal portion of the casing (660) that is proximal to the nozzle (678),
the nozzle (678) is oriented to direct the sample from the outlet (668) distally toward the rim of the pool (682), and
the sample pad (702) is disposed below the nozzle axis.

6. The apparatus (600) according to claim 5, wherein the chamber (680) has a hollow volume, and most of the hollow volume is disposed above the nozzle axis.

7. The apparatus (600) according to claim 5, wherein the floor (684) slopes distally upward.

8. The apparatus (600) according to claim 7, wherein a proximal part of the floor (684) is disposed below the nozzle axis, and the floor (684) slopes distally upward to intersect the nozzle axis.

9. The apparatus (600) according to claim 7, wherein the floor (684) slopes distally upward at 1-10 degrees with respect to the nozzle axis.

10. The apparatus (600) according to claim 5, wherein the channel (664) is colinear with the nozzle (678).

11. The apparatus (600) according to claim 5, wherein the sample pad (702) is parallel with the nozzle axis.

12. The apparatus (600) according to any one of claims 1-11, wherein the casing (660) further defines a spillway (694), and the floor (684) further defines one or more overflow holes (692) therethrough, the one or more overflow holes (692) and the spillway (694) configured to direct excess sample out of the chamber (680) via the one or more overflow holes (692) to the spillway (694).

13. The apparatus (600) according to claim 12, wherein the spillway (694) defines a drainage hole, and wherein the drainage hole is disposed proximally of the sample pad.

14. The apparatus (600) according to claim 12, wherein at least part of the spillway (694) is disposed below at least part of the lateral flow platform (700).

15. The apparatus (600) according to claim 12, wherein the one or more overflow holes (692) are disposed proximally from the aperture (686).

## Patentansprüche

1. Vorrichtung (600) zur Verwendung durch ein Subjekt, wobei die Vorrichtung Folgendes umfasst:
einen Probenehmer (120), umfassend:
einen Schwamm (130), der dazu konfiguriert ist, in einem Mund des Subjekts platziert zu werden und eine Speichelprobe aus dem Mund zu absorbieren;
einen Kolben (122), der einen distalen Abschnitt (124) aufweist, der an den Schwamm (130) gekoppelt ist;
eine Lateralflussplattform (700), die ein Probenkissen (702) umfasst; und
ein Gehäuse (660), das die Lateralflussplattform (700) aufnimmt und Folgendes umfasst:
einen Kanal (664), aufweisend:
eine Öffnung (666) in den Kanal (664) an einer proximalen Region des Kanals (664), und
einen Auslass (668) aus dem Kanal (664) an einer distalen Region des Kanals (664), wobei der Probenehmer (120) dazu konfiguriert ist, während der Schwamm (130) die Probe hält, den Schwamm (130) in den Kanal (664) über die Öffnung (666) einzuführen, wobei der Kolben (122) dazu bemessen ist, eng anliegend distal innerhalb des Kanals (664) in einer Weise zu gleiten, die den Schwamm (130) komprimiert und die Probe aus dem Schwamm (130) und durch den Auslass (668) drückt;
**dadurch gekennzeichnet, dass** das Gehäuse (660) ferner Folgendes umfasst:
eine Kammer (680), die einen Boden (684) aufweist, wobei der Boden (684) eine Apertur (686) dort hindurch definiert, wobei zumindest ein Abschnitt des Probenkissens (702) unter der Apertur (686) vorgesehen ist, sodass die Apertur (686) und der Abschnitt des Probenkissens (702) gemeinsam einen Pool (682) definieren, wobei die Apertur (686) einen Rand des Pools (682) definiert und der Abschnitt des Probenkissens (702) einen Boden des Pools (682) definiert; und
eine Düse (678), die sich von dem Auslass (668) erstreckt und sich in die Kammer (680) in einer Orientierung öffnet, welche die Probe von dem Auslass (668) zu dem Rand des Pools (682) leitet.

2. Vorrichtung (600) nach Anspruch 1, wobei das Gehäuse (660) dazu konfiguriert ist, zu ermöglichen, dass erhöhter Druck in der Kammer (680), der daraus resultiert, dass der Kolben (122) eng anliegend distal innerhalb des Kanals (664) gleitet, nach außerhalb des Gehäuses (660) freigegeben wird.

3. Vorrichtung (600) nach Anspruch 1, ferner umfassend (i) einen porösen Träger (170), der innerhalb des Kanals (664) proximal von dem Auslass (668) vorgesehen ist, und (ii) eine oder mehrere trockene Pufferkomponenten, die in dem Träger (170) gehalten werden, wobei zumindest eine der Pufferkomponenten ein Tensid (172) ist, das in dem Träger (170) gehalten wird, wobei:
der Kolben (122) dazu bemessen ist, eng anliegend distal innerhalb des Kanals in einer Weise zu gleiten, die den Schwamm komprimiert und die Probe drückt:
aus dem Schwamm (130) und in den Träger (170), wodurch zumindest etwas der Pufferkomponenten gelöst wird, und
aus dem Träger (170) mit den gelösten Pufferkomponenten und durch den Auslass (668),
wobei der Träger (170) eine Vielzahl von Fasern (174) umfasst, die angeordnet ist, sodass:
Räume zwischen den Fasern (174) Poren (178) des Trägers (170) definieren, und
die Fasern (174) im Allgemeinen nicht entlang einer Längsachse des Trägers (170) angeordnet sind.

4. Vorrichtung (600) nach einem der Ansprüche 1-3, wobei die Vorrichtung kein Albumin umfasst.

5. Vorrichtung (600) nach einem der Ansprüche 1-4, wobei:
die Düse (678) eine Düsenachse definiert,
der Kanal (664) an einem proximalen Abschnitt des Gehäuses (660) vorgesehen ist, der proximal zu der Düse (678) ist,
die Düse (678) dazu orientiert ist, die Probe von dem Auslass (668) distal zu dem Rand des Pools (682) zu leiten, und
das Probenkissen (702) unter der Düsenachse vorgesehen ist.

6. Vorrichtung (600) nach Anspruch 5, wobei die Kammer (680) ein Hohlvolumen aufweist und das meiste des Hohlvolumens über der Düsenachse vorgesehen ist.

7. Vorrichtung (600) nach Anspruch 5, wobei sich der Boden (684) distal nach oben neigt.

8. Vorrichtung (600) nach Anspruch 7, wobei ein proximaler Teil des Bodens (684) unter der Düsenachse vorgesehen ist und sich der Boden (684) distal nach oben neigt, um die Düsenachse zu schneiden.

9. Vorrichtung (600) nach Anspruch 7, wobei sich der Boden (684) distal nach oben mit 1-10 Grad in Bezug auf die Düsenachse neigt.

10. Vorrichtung (600) nach Anspruch 5, wobei der Kanal (664) kolinear mit der Düse (678) ist.

11. Vorrichtung (600) nach Anspruch 5, wobei das Probenkissen (702) parallel mit der Düsenachse ist.

12. Vorrichtung (600) nach einem der Ansprüche 1-11, wobei das Gehäuse (660) ferner einen Abfluss (694) definiert und der Boden (684) ferner ein oder mehrere Überflusslöcher (692) dort hindurch definiert, wobei das eine oder die mehreren Überflusslöcher (692) und der Abfluss (694) dazu konfiguriert sind, überschüssige Probe aus der Kammer (680) über das eine oder die mehreren Überflusslöcher (692) zu dem Abfluss (694) zu leiten.

13. Vorrichtung (600) nach Anspruch 12, wobei der Abfluss (694) ein Drainageloch definiert und wobei das Drainageloch proximal zu dem Probenkissen vorgesehen ist.

14. Vorrichtung (600) nach Anspruch 12, wobei zumindest Teil des Abflusses (694) unter zumindest Teil der Lateralflussplattform (700) vorgesehen ist.

15. Vorrichtung (600) nach Anspruch 12, wobei das eine oder die mehreren Überflusslöcher (692) proximal von der Apertur (686) vorgesehen sind.

## Revendications

1. Appareil (600), destiné à être utilisé par un sujet, l'appareil comprenant :
un échantillonneur (120), comprenant :
une éponge (130), conçue pour être placée dans la bouche du sujet et pour absorber un échantillon de salive de la bouche ;
un piston (122), possédant une partie distale (124) qui est couplée à l'éponge (130) ;
une plateforme à écoulement latéral (700), comprenant un tampon d'échantillon (702) ; et
un boîtier (660), logeant la plateforme à écoulement latéral (700), et comprenant :
un canal (664), comportant :
un orifice (666) dans le canal (664) au niveau d'une zone proximale du canal (664), et
une sortie (668) du canal (664) au niveau d'une zone distale du canal (664), l'échantillonneur (120) étant conçu pour, pendant que l'éponge (130) maintient l'échantillon, introduire l'éponge (130) dans le canal (664) par l'intermédiaire de l'orifice (666), le piston (122) étant dimensionné de manière à coulisser bien ajusté de façon distale à l'intérieur du canal (664) d'une manière qui comprime l'éponge (130) et pousse l'échantillon hors de l'éponge (130) et à travers la sortie (668) ;
**caractérisé en ce que** le boîtier (660) comprend en outre :
une chambre (680) comportant un sol (684), le sol (684) définissant une ouverture (686) à travers celui-ci, au moins une partie du tampon d'échantillon (702) étant disposée sous l'ouverture (686) de sorte que l'ouverture (686) et la partie du tampon d'échantillon (702) définissent collectivement un réservoir (682), l'ouverture (686) définissant un bord du réservoir (682), et la partie du tampon d'échantillon (702) définissant le fond du réservoir (682) ; et
une buse (678), s'étendant depuis la sortie (668), et s'ouvrant dans la chambre (680) dans une orientation qui dirige l'échantillon de la sortie (668) vers le bord du réservoir (682).

2. Appareil (600) selon la revendication 1, ledit boîtier (660) étant conçu pour permettre à une pression élevée dans la chambre (680), résultant du coulissement bien ajusté du piston (122) de façon distale à l'intérieur du canal (664), d'être libérée à l'extérieur du boîtier (660).

3. Appareil (600) selon la revendication 1, comprenant en outre (i) un support poreux (170), disposé à l'intérieur du canal (664) de manière proximale à partir de la sortie (668), et (ii) un ou plusieurs composants tampons secs, maintenus dans le support (170), au moins l'un des composants tampons étant un tensioactif (172) maintenu dans le support (170) :
ledit piston (122) étant dimensionné de manière à coulisser bien ajusté de façon distale à l'intérieur du canal d'une manière qui comprime l'éponge et pousse l'échantillon :
hors de l'éponge (130) et dans le support (170), en dissolvant au moins certains des composants du tampon, et
hors du support (170) avec les composants du tampon dissous, et à travers la sortie (668),
ledit support (170) comprenant une pluralité de fibres (174) agencées de sorte que :
des espaces entre les fibres (174) définissent des pores (178) du support (170), et
les fibres (174) ne soient généralement pas agencées le long d'un axe longitudinal du support (170).

4. Appareil (600) selon l'une quelconque des revendications 1 à 3, ledit appareil ne comprenant pas d'albumine.

5. Appareil (600) selon l'une quelconque des revendications 1 à 4 :
ladite buse (678) définissant un axe de buse,
ledit canal (664) étant disposé au niveau d'une partie proximale du boîtier (660) qui est proximale par rapport à la buse (678),
ladite buse (678) étant orientée de manière à diriger l'échantillon de la sortie (668) de façon distale vers le bord du réservoir (682), et
ledit tampon d'échantillon (702) étant disposé sous l'axe de la buse.

6. Appareil (600) selon la revendication 5, ladite chambre (680) présentant un volume creux, et la majeure partie du volume creux étant disposée au-dessus de l'axe de la buse.

7. Appareil (600) selon la revendication 5, ledit sol (684) étant incliné de façon distale vers le haut.

8. Appareil (600) selon la revendication 7, une partie proximale du sol (684) étant disposée sous l'axe de la buse, et ledit sol (684) étant incliné de façon distale vers le haut pour couper l'axe de la buse.

9. Appareil (600) selon la revendication 7, ledit sol (684) étant incliné de façon distale vers le haut suivant un angle de 1 à 10 degrés par rapport à l'axe de la buse.

10. Appareil (600) selon la revendication 5, ledit canal (664) étant colinéaire avec la buse (678).

11. Appareil (600) selon la revendication 5, ledit tampon d'échantillon (702) étant parallèle à l'axe de la buse.

12. Appareil (600) selon l'une quelconque des revendications 1 à 11, ledit boîtier (660) définissant en outre un déversoir (694), et ledit sol (684) définissant en outre un ou plusieurs trous de débordement (692) en son travers, lesdits un ou plusieurs trous de débordement (692) et ledit déversoir (694) étant conçus pour diriger l'échantillon en excès hors de la chambre (680) par l'intermédiaire desdits un ou plusieurs trous de débordement (692) vers ledit déversoir (694).

13. Appareil (600) selon la revendication 12, ledit déversoir (694) définissant un trou de drainage, et ledit trou de drainage étant disposé de façon proximale par rapport au tampon d'échantillon.

14. Appareil (600) selon la revendication 12, au moins une partie du déversoir (694) étant disposée sous au moins une partie de la plateforme d'écoulement latéral (700).

15. Appareil (600) selon la revendication 12, lesdits un ou plusieurs trous de débordement (692) étant disposés de façon proximale par rapport à l'ouverture (686).
